(19) Europäisches Patentamt
European Patent Office
Office européen des brevets

(11) **EP 3 567 362 B1**

(12) **EUROPÄISCHE PATENTSCHRIFT**

(45) Veröffentlichungstag und Bekanntmachung des Hinweises auf die Patenterteilung:
**30.03.2022   Patentblatt 2022/13**

(21) Anmeldenummer: **18171588.9**

(22) Anmeldetag: **09.05.2018**

(51) Internationale Patentklassifikation (IPC):
**G01N 1/28** (2006.01)          **G01N 33/49** (2006.01)

(52) Gemeinsame Patentklassifikation (CPC):
**G01N 1/286;** G01N 33/49; G01N 2001/2886

(54) **VERFAHREN UND VORRICHTUNG ZUR SEGMENTIERUNG UND VEREINZELUNG FLÄCHIG VERTEILTER PROBEN MITTELS LASERSCHNEIDENS**

METHOD AND DEVICE FOR SEGMENTING AND SEPARATING FLAT DISTRIBUTED SAMPLES USING LASER CUTTING

PROCÉDÉ ET DISPOSITIF DE SEGMENTATION ET DE SÉPARATION DES ÉCHANTILLONS RÉPARTIS EN SURFACE PAR DÉCOUPE LASER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**13.11.2019   Patentblatt 2019/46**

(73) Patentinhaber: **Euroimmun Medizinische Labordiagnostika AG
23560 Lübeck (DE)**

(72) Erfinder:
• **Stöcker, Winfried
23627 Groß Grönau (DE)**
• **Pannhoff, Helge
20535 Hamburg (DE)**
• **Krause, Steffen
23566 Lübeck (DE)**

(56) Entgegenhaltungen:
WO-A1-2014/053955          WO-A1-2014/096055
WO-A2-2006/031574          WO-A2-2009/068749
WO-A2-2010/081171          DE-A1-102014 203 656
DE-A1-102014 203 747      US-A1- 2003 227 611
US-A1- 2015 040 688

• Nichia: "Blue Laser Diode - NDB4116", , 2. Oktober 2012 (2012-10-02), XP055517658, Gefunden im Internet: URL:http://www.nichia.co.jp/en/product/laser.html [gefunden am 2018-10-22]

EP 3 567 362 B1

**Beschreibung**

Die Erfindung betrifft ein Verfahren und eine Vorrichtung zur Vereinzelung und Segmentierung flächig verteilter Patientenproben mittels Laserschneidens.

Bekannt sind flächige Probenträger, welche eine flüssigkeitsadsorbierende Stoffschicht aufweisen, beispielsweise auf Baumwollbasis. Auf die flüssigkeitsadsorbierenden Stoffschicht kann eine flüssige Patientenprobe wie beispielsweise Blut aufgebracht werden, der sich in der Stoffschicht flächig verteilt und antrocknet, sodass beispielsweise ein sogenannter "Dried Blood Spot" (DBS) ausgebildet wird. Nach Antrocknen der Patientenprobe in der Stoffschicht kann dann der Probenträger mitsamt der angetrockneten Probe für eine spätere biochemische Analyse zu einem Labor verbracht werden. Zum Zwecke der biochemischen Analyse wird dort ein Segment, in welchem sich zumindest ein Teil der angetrockneten Patientenprobe befindet, aus dem Probenträger herausgetrennt bzw. vereinzelt. Ein solches vereinzeltes Segment mitsamt eines Teils der Patientenprobe kann dann beispielsweise einer PCR (polymerase chain reaction) zugeführt werden.

Die Proben werden in flüssiger Form aufgetragen und können dann auf der Oberfläche bzw. in der Stoffschicht antrocknen, bevor die Segmente vereinzelt werden. Alternativ bleiben die Proben aber flüssig und werden nach der Vereinzelung des Segmentes mit dem Segement als flüssige Probe auf einem oder mehreren Labor-Arbeitsplätzen weiterverarbeitet und analysiert. Insofern eignet sich die Erfindung auch für die Verteilung flüssiger Proben im Labor.

Zum Zwecke einer solchen Vereinzelung eines Segmentes von einem Probenträger sind sogenannte Stanzverfahren bekannt, bei welchen mittels eines Stanzmessers das Segment aus dem Probenträger herausgestanzt wird und dann ggf. mit einem Stempel aus dem Stanzmesser herausgedrückt wird, woraufhin das Segment in ein Analysebehältnis herabfällt.

Hierbei kann es vorkommen, dass sich das Segment elektrostatisch auflädt und nicht in das Behältnis herabfällt. Ferner kann es vorkommen, dass durch den Stanzvorgang ein Teil der Probe an dem Stanzmesser und/oder dem Stempel hängenbleibt und nachfolgende Proben kontaminiert, was im Falle einer PCR zu Fehlbestimmungen führen kann.

Erfolgt die Vereinzelung von Segmenten mittels eines Stanzmessers, so erfährt das Stanzmesser durch die Stanzvorgänge einen mechanischen Abrieb bzw. unterliegt einer mechanischen Abnutzung, sodass es von Zeit zu Zeit ausgetauscht werden muss.

Bekannt sind ferner aus der US 2015/0040688 A1 sowie der WO 2009/068749 A2 Verfahren, bei welchen sogenannte "Dried Blood Spots" aus einem Probenträgermaterial mittels eines Laserstrahls ausgeschnitten werden. Die WO 2009/068749 A2 verweist hierbei lediglich auf die Möglichkeit, unterschiedliche Laserarten zu verwenden. Aus der WO 01/31317 A1 ist es offenbart, explizit einen Kohlenstoffdioxid-Laser zu verwenden.

Erfindungsgemäß wird ein Verfahren zur Segmentierung und Vereinzelung flächig verteilter Patientenproben vorgeschlagen. Hierbei weist ein Probenträger eine Stoffschicht auf, die geeignet ist, eine flüssige Patientenprobe zu adsorbieren. Der Probenträger weist in oder auf einem zu vereinzelnden Segment zumindest einen Teil der Patientenprobe auf. Das kann ein noch flüssiger Probentropfen sein, welcher vorzugsweise Blut aufweist. Alternativ kann dies ein getrockneter Probentropfen, vorzugsweise ein "Dried Blood Spot" (getrockneter Blutstropfen). Mittels einer Lasereinheit wird ein Laserstrahl erzeugt, welcher dann entlang zumindest eines Teils einer Randkurve des zu vereinzelnden Segmentes geführt wird, um das Segment von dem Probenträger abzuschneiden. Hierbei wird eine solche Lasereinheit verwendet, welche einen Laserstrahl mit einer Wellenlänge in einem Bereich von 440 bis 455 nm aufweist.

Die erfindungsgemäße Wahl des Wellenlängenbereiches ist insbesondere in dem Fall vorteilhaft, dass die Patientenprobe Blut aufweist, da dann Laserlicht einer Wellenlänge aus dem hier vorgeschlagenen Bereich von 380 bis 600 nm besonders gut durch die Kombination aus Probenträgermaterial und Blut absorbiert wird. Hierbei befindet sich entlang der Randkurve des Segmentes ein Teil der Patientenprobe, welche Blut aufweist. Aufgrund der Kombination aus Probenträgermaterial und Blut kommt es zu einer erhöhten Absorption der Energie des Laserlichtes, sodass ein Energieeintrag des Laserlichtes zum Schneiden des Segmentes entlang der Randkurve optimiert wird. Hierdurch werden eine reduzierte Schnittzeit und ein verbessertes, genaueres Schnittergebnis erreicht.

Die Lasereinheit ist ein Halbleiterlaser, wobei der Laserstrahl Wellenlängen im Bereich von 440 bis 455 nm aufweist. Eine Erzeugung von Laserlicht mit einer gewissen optischen Mindestleistung von mehr als 1 Watt, vorzugsweise mehr als 2 Watt, kann besonders kostengünstig mittels eines Halbleiterlasers erzeugt werden, welcher eine Wellenlänge in dem hier angegebenen Bereich von 440 bis 455 nm aufweist. Halbleiterlaser außerhalb des Wellenlängenbereiches von 440 bis 450 nm erzeugen bei gleichem Kostenaufwand Laserstrahlen mit einer optischen Leistung, welche eindeutig unterhalb von 1 Watt liegen. Derartige Leistungswerte unterhalb von 1 Watt erlauben dann kein zuverlässiges Schneiden des Probenträgermaterials mehr. Daher ist die Auswahl des Wellenlängenbereiches von 440 bis 455 nm besonders vorteilhaft, da in diesem Bereich Schnittergebnisse mit hinreichender Qualität bei vertretbarem Kostenaufwand für den Halbleiterlaser erreicht werden können. Ferner ist ein Halbleiterlaser von deutlich kleinerer Baugröße als ein Kohlenstoffdioxid-Laser. Erst durch eine optische Leistung von mehr als 1 Watt wird es möglich, in Laboren üblicherweise verwendetes Probenträgermaterial zuverlässig zu schneiden. Kohlenstoffdioxid-Laser besitzen ein relatives großes Bauvolumen und verursachen gewisse Mindestkosten. Ferner ist für ein Betreiben eines Kohlenstoffdioxid-Lasers eine Wasserkühlung und ein Hochspannungsnetzeil erforderlich, welches zu einem komplexen und aufwändigen Gesamtaufbau führt.

Vorzugsweise ist der Probenträger derart ausgestaltet, dass dieser Aussparungen bzw. Löcher aufweist, sodass das zu vereinzelnde Segment mit dem Rest des Probenträgers durch einen oder mehrere Stege verbunden ist, vorzugsweise nur durch einen oder mehrere Stege verbunden ist. Es muss also der Laserstrahl lediglich entlang jener Teilbereiche der Randkurve des Segmentes geführt werden, an welchen sich die Stege befinden, um das Segment vollständig aus dem Probenträger herauszuschneiden. Wird eine Patientenprobe wie z.B. Blut auf ein Segment eines Probenträgers aufgetropft, so verteilt sich diese flächig über den Segmentbereich und kann dann auch über den einen oder die mehreren Stege hin zu dem übrigen Bereich des Probenträgers fließen. Durch die Stege wird es ermöglicht, dass im Bereich des Segmentes eine definierte Probenmenge bzw. ein definiertes Volumen an Probenmaterial verbleibt und dass überschüssiges Probenmaterial hin zu dem restlichen Probenträger ablaufen kann. Ferner wird es durch die Stege ermöglicht, dass der Laserstrahl nicht entlang der vollständigen Randkurve geführt werden muss, um das Segment aus dem Probenträger herauszuschneiden, sondern dass es genügt, den Laserstrahl lediglich entlang jener Teilbereiche der Randkurve zu führen, an welchen sich die Stege befinden. Dieses ermöglicht eine weitere Reduktion der Schnittzeit.

Vorzugsweise weist der Probenträger eine flächig durchgängige Folienschicht mit einer Thermoplastfolie auf, wobei das Segment oberhalb der Folienschicht in einer Stoffschicht ein Stoffschichtsegment aufweist, welches auf der Thermoplastfolie aufgebracht ist und welches durch die Randkurve begrenzt ist. Diese Ausgestaltung ist vorteilhaft, da während des Laserschneidens entlang der Randkurve lediglich die Thermoplastfolie durch den Laserstrahl durchtrennt werden muss, nicht aber eine vollständige Stoffschicht. Da eine Thermoplastfolie durch einen Laserstrahl einfacher zu durchtrennen ist als eine flüssigkeitsadsorbierende Stoffschicht eines Probenträgers, wird hierdurch die Schnittzeit weiter reduziert.

Vorzugsweise wird ein Luftstrom hin zu einer Schnittstelle des Laserstrahls mit dem Probenträger geführt und es wird zumindest ein Teil des Luftstromes von der Schnittstelle abgesaugt. Dieses ist vorteilhaft, da hierdurch eine thermische Beeinflussung des Probenträgers und der darin enthaltenen Probe durch den Laserstrahl auf die tatsächliche Schnittstelle begrenzt wird. Dieses reduziert insbesondere eine thermische Beeinflussung der Probe in Bereichen nahe zur Randkurve, sodass eine Veränderung der Probe durch solch eine thermische Beeinflussung minimiert wird.

Vorzugsweise werden mittels mehrerer Teil-Lasereinheiten jeweilige Laserstrahlen erzeugt, welche mittels wenigstens einer Optikeinheit an jeweilige Schnittpunkte auf der Randkurve gelenkt werden, sodass mittels eines gleichzeitigen Führens der jeweiligen Laserstrahlen entlang jeweiliger Teilabschnitte der Randkurve des Segmentes das Segment von dem Probenträger abgeschnitten wird. Hierdurch wird abermals die Schnittzeit reduziert, da beispielsweise im Falle einer kreisförmigen Randkurve bei Verwendung von drei Teil-Lasereinheiten jede der Teil-Lasereinheiten nur jeweils einen Winkelbereich von 120° überstreichen muss, wobei jede der Teil-Lasereinheiten dies vorzugsweise gleichzeitig durchführen kann, so dass gegenüber einer Verwendung einer einzelnen Lasereinheit und Überstreichen eines Winkelbereiches von 360° die Schnittzeit auf 1/3 reduziert werden kann. In dem Fall, dass der Probenträger zuvor genannte Aussparungen aufweist, kann durch Verwendung der Teil-Lasereinheiten dann eine noch stärkere Reduktion der Schnittzeit zur Vereinzelung des Segmentes erzielt werden. Weist das Segment beispielsweise einen Durchmesser von ca. 4,75 mm auf und somit eine Randkurve des Segmentes einen Umfang von ca. 15 mm, so kann mittels einer einzelnen Lasereinheit bzw. eines einzelnen Halbleiterlasers ein Segment ohne Aussparungen in einer Zeit von 6 Sekunden durchgeschnitten werden, wie später noch im Beispiel 1 dargelegt. Werden jedoch drei Teil-Lasereinheiten bzw. drei Halbleiterlaser gleichzeigt verwendet und sind ferner Aussparungen so vorhanden, dass nur drei Stege einer jeweiligen Breite von ca. 1,6 mm vorhanden sind, und werden die drei jeweiligen Teil-Lasereinheiten so geführt, dass diese gleichzeitig jeweils einen jeweiligen der drei Stege durchschneiden, so reduziert sich die Schnittzeit auf ca.

$$(6 \text{ Sekunden} * 1,6 / 15) = 0,64 \text{ Sekunden} ,$$

welches eine Reduktion um einen ungefähren Faktor von 0,106 entspricht.

Vorzugsweise werden mittels der Optikeinheit die jeweiligen Laserstrahlen an die jeweiligen, unterschiedlichen Schnittpunkte auf der Randkurve so gelenkt, dass sich für die jeweiligen Laserstrahlen in einer optischen Ebene, welche mit dem Probenträger zusammenfällt, jeweilige Fokuspunkte ergeben. Vorzugsweise wird die Optikeinheit so eingestellt, dass ein Abstand der jeweiligen Schnittpunkte der jeweiligen Laserstrahlen in der optischen Ebene auf dem Probenträger zueinander so eingestellt wird, so dass sich für unterschiedliche Abstände der jeweiligen Schnittpunkte in der optischen Ebene auf dem Probenträger entsprechende, jeweilige Fokuspunkte der Laserstahlen ergeben. Vorzugsweise weist die Optikeinheit wenigstens zwei optische Linsen auf. Durch

- Einstellen eines Abstandes einer ersten optischen Linse der zwei optischen Linsen zu einer zweiten optischen Linse der zwei optischen Linsen zueinander

- sowie ferner Einstellen eines Abstandes der ersten optischen Linse zu den Teil-Lasereinheiten

erfolgt dann das Einstellen des Abstandes der jeweiligen Schnittpunkte auf dem Probenträger. Dieses ist vorteilhaft, da für verschiedene Segmentgrößen verschiedener Arten von Probenträgern mittels der Einstellung der optischen Einheit bzw. deren Linsen die Teil-Lasereinheiten nicht in ihrer Position gegenüber dem Probenträger verändert werden müssen, um bei einer zu erreichenden Änderung jeweiliger Abstände der jeweiligen Schnittpunkte trotzdem eine Fokussierung der jeweiligen Laserstrahlen in der optischen Ebene bzw. der Ebene des Probenträgers zu erreichen.

Vorgeschlagen wird ferner eine Vorrichtung zur Vereinzelung und Segmentierung flächig verteilter Patientenproben mittels Laserschneidens. Die Vorrichtung weist eine Halterung zum Halten eines Probenträgers auf sowie eine Lasereinheit zum Erzeugen eines Laserstrahls. Ferner weist die Vorrichtung wenigstens ein Positionierungsmittel zum Ändern einer Position des Laserstrahls in Relation zu dem Probenträger auf. Die Vorrichtung weist wenigstens eine Steuereinheit zum Ansteuern des wenigstens einen Positionierungsmittels auf. Die Steuereinheit ist ausgebildet, das wenigstens eine Positionierungsmittel derart anzusteuern, dass der Laserstrahl entlang zumindest eines Teils einer Randkurve eines Segmentes des Probenträgers geführt wird, sodass das Segment von dem Probenträger abgeschnitten wird. Die Wellenlänge des Laserstrahls liegt hierbei in einem Bereich von 440 bis 455 nm. Die untere Grenze des Wellenlängenbereiches beträgt also 440 nm, die obere Grenze des Wellenlängenbereiches beträgt also 455 nm. In einem Beispiel, das nicht Teil der Erfindung ist, beträgt die untere Grenze des Wellenlängenbereiches 390 nm oder 400 nm oder 410 nm oder 420 nm oder 430 nm. In einem Beispiel, das nicht Teil der Erfindung ist, beträgt die obere Grenze des Wellenlängenbereiches 590 nm oder 580 nm oder 570 nm oder 560 nm oder 550 nm oder 540 nm oder 530 nm oder 520 nm oder 510 nm oder 500 nm oder 490 nm oder 480 nm oder 470 nm oder 460 nm.

Die Lasereinheit ist ein Halbleiterlaser, wobei der Laserstrahl eine Wellenlänge im Bereich von 440 bis 455 nm aufweist.

Vorzugsweise weist der Probenträger Aussparungen auf, sodass das Segment mit dem Rest des Probenträgers durch einen oder mehrere Stege verbunden ist, vorzugsweise nur durch einen oder mehrere Stege verbunden ist. Hierbei ist die Steuereinheit ausgebildet, das Positionierungsmittel derart anzusteuern, dass der Laserstrahl entlang jener Teilbereiche der Randkurve des Segmentes geführt wird, an welchen sich die Stege befinden.

Vorzugsweise weist die Vorrichtung eine Luftfördereinheit zum Zuführen eines Luftstromes hin zu einer Schnittstelle des Laserstrahls mit dem Probenträger auf. Vorzugsweise weist die Vorrichtung ferner eine Ablufteinheit zum Absaugen zumindest eines Teils des Luftstromes von der Schnittstelle weg.

Vorzugsweise weist die Vorrichtung

- mehrere, jeweilige Teil-Lasereinheiten zum Erzeugen jeweiliger Laserstrahlen,
- sowie eine Optikeinheit zum Lenken der jeweiligen Laserstrahlen an jeweilige Schnittpunkte auf der Randkurve,

auf und ist ferner dadurch gekennzeichnet,

- dass das Positionierungsmittel geeignet ist, jeweilige Positionen der jeweiligen Laserstrahlen in Relation zu dem Probenträger zu ändern,
- und dass die Steuereinheit ausgebildet ist, das Positionierungsmittel derart anzusteuern, dass die jeweiligen Laserstrahlen gleichzeitig entlang jeweiliger Teilabschnitte der Randkurve geführt werden, sodass das Segment von dem Probenträger abgeschnitten wird.

Vorzugsweise ist die Optikeinheit ausgebildet, die jeweiligen Laserstrahlen an die jeweiligen, unterschiedlichen Schnittpunkte auf der Randkurve so zu lenken, dass sich für die jeweiligen Laserstrahlen in einer optischen Ebene, welche mit dem Probenträger zusammenfällt, jeweilige Fokuspunkte ergeben. Hierbei ist die Optikeinheit so einstellbar, dass ein Abstand der jeweiligen Schnittpunkte der jeweiligen Laserstrahlen in der optischen Ebene auf dem Probenträger zueinander so eingestellt werden kann, dass sich auch für unterschiedliche Abstände der jeweiligen Schnittpunkte in der optischen Ebene auf dem Probenträger entsprechende, jeweilige Fokuspunkte der Laserstahlen ergeben. Vorzugsweise ist hierbei die Steuereinheit ausgebildet ist, die Optikeinheit mittels eines Steuersignals zur Veränderung bzw. Einstellung des Abstandes der jeweiligen Schnittpunkte in der optischen Ebene auf dem Probenträger anzusteuern.

Vorzugsweise weist die Optikeinheit wenigstens zwei optische Linsen auf, wobei die Steuereinheit ferner ausgebildet ist,

- einen Abstand einer ersten optischen Linse zu einer zweiten optischen Linse
- sowie ferner einen Abstand der ersten optischen Linse zu den Teil-Lasereinheiten einzustellen, so dass ein Abstand der jeweiligen Schnittpunkte der jeweiligen Laserstrahlen mit dem Probenträger eingestellt wird.

Es kann zusammenfassend gesagt werden, dass die Erfindung ein Verfahren und eine Vorrichtung zur Vereinzelung eines Segmentes eines flächigen Probenträgers mittels Laserschneidens betrifft. Hierbei weist der Probenträger eine Stoffschicht auf, welche geeignet ist, eine flüssige Patientenprobe zu adsorbieren, vorzugsweise basierend auf einer Kapillarwirkung. Die Stoffschicht weist vorzugsweise eine flächige Faserstruktur auf, welche flüssigkeitsadsorbierend ist, sodass beim Aufbringen einer Patientenprobe auf der Stoffschicht eine flächige Verteilung der Patientenprobe in der Stoffschicht durch Kapillarwirkung bewirkt wird. Die Stoffschicht ist vorzugsweise auf Baumwollbasis hergestellt. Besonders bevorzugt weist die Stoffschicht Baumwollflies auf. Vorzugsweise beträgt der Baumwollanteil der Stoffschicht mehr als 80% Baumwolle, besonders bevorzugt mehr als 95 % Baumwolle. Vorzugsweise besteht der Probenträger aus der flächig ausgeprägten Stoffschicht.

Vorteilhafte Ausführungsformen der Erfindung sind Gegenstand der abhängigen Ansprüche und werden in der folgenden Beschreibung unter teilweise Bezugnahme auf die Figuren näher erläutert.

Dabei zeigen

Figur 1 eine Aufsicht einer ersten Ausführungsform eines Probenträgers,
Figur 2 eine Seitenansicht einer zweiten Ausführungsform eines Probenträgers,
Figur 3 eine Aufsicht einer dritten Ausführungsform eines Probenträgers,
Figur 4 ein Diagramm zur Darstellung hinsichtlich einer Remission von Laserlicht durch einen Probenträger in Abhängigkeit einer Wellenlänge des Laserlichtes,
Figur 5 und 6 eine Aufsicht bzw. eine Seitenansicht einer vierten Ausführungsform eines Probenträgers,
Figur 7 eine bevorzugte Ausführungsform der erfindungsgemäßen Vorrichtung,
Figuren 8 und 9 bevorzugte Ausgestaltungen von Probenträgern der vierten Ausführungsform,
Figur 10a und 10b eine Aufsicht bzw. eine Seitenansicht einer bevorzugten Anordnung von Teil-Lasereinheiten,
Figuren 11a und 11b jeweilige Darstellungen von Strahlengängen im Falle unterschiedlich positionierter optischer Linsen,
Figuren 12a bis 12c jeweilige Abläufe jeweiliger Verfahrensschritte,
Figur 13 Beispiele vereinzelter Segmente,
Figuren 14a und 14b Analyseergebnisse für eine Analyse bezüglich Anti-Röteln IgG,
Figuren 15a und 15b Analyseergebnisse für eine Analyse bezüglich Vitamin D.

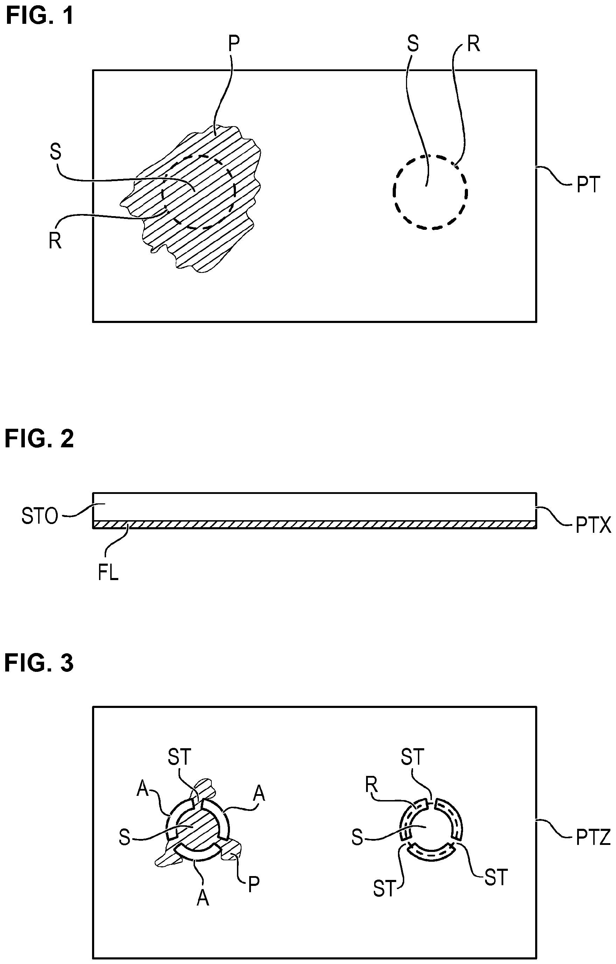

Figur 1 zeigt eine Aufsicht einer ersten Ausführungsform eines Probenträgers PT, welcher Segmente S aufweist, für welche jeweilige Randkurven R skizziert sind. Die dargestellten Randkurven R repräsentieren in dieser Ausführungsform keine Perforation oder Durchdringung des Probenträgers PT, sondern stellen lediglich eine Indikation eines imaginären äußeren Randes bzw. einer imaginären Randkurve R eines zu vereinzelnden Segmentes S dar. Im Bereich des linksseitigen Segmentes S befindet sich eine Patientenprobe P, vorzugsweise in Form von flüssigem Blut oder in Form eines angetrockneten Blutstropfens. Bei Auftropfen einer Patientenprobe P auf den Probenträger verteilt sich die Patientenprobe P aufgrund der Kapillarwirkung auf und in einer Stoffschicht des Probenträgers flächig sowohl im Bereich des Segmentes S als auch über die Randkurve R des Segmentes S hinausgehend. Hierbei weist der Probenträger eine Stoffschicht auf, welche geeignet ist, eine flüssige Patientenprobe zu adsorbieren, vorzugsweise basierend auf einer Kapillarwirkung. Die Stoffschicht weist vorzugsweise eine flächige Faserstruktur auf, welche flüssigkeitsadsorbierend ist, sodass beim Aufbringen einer Patientenprobe auf der Stoffschicht eine flächige Verteilung der Patientenprobe in der Stoffschicht durch Kapillarwirkung bewirkt wird. Die Stoffschicht ist vorzugsweise auf Baumwollbasis hergestellt. Besonders bevorzugt weist die Stoffschicht Baumwollflies auf. Vorzugsweise beträgt der Baumwollanteil der Stoffschicht mehr als 80% Baumwolle, besonders bevorzugt mehr als 95 % Baumwolle. Besonders bevorzugt besteht der Probenträger aus der flächig ausgeprägten Stoffschicht.

Die Figur 2 zeigt eine zweite mögliche Ausführungsform eines Probenträgers PTX, bei welchem zusätzlich zu einer Stoffschicht STO unterhalb der Stoffschicht STO eine Folienschicht FL vorhanden ist. Die Folienschicht FL kann eine mechanische Stabilität des Probenträgers PTX erhöhen. Die Ausführungsformen des Probenträges PT aus der Figur 1 sowie des Probenträger PTX aus der Figur 2 haben gemein, dass sie beide eine zuvor beschriebene Stoffschicht STO aufweisen.

Wird ein Probenträger PT aus der Figur 1 bereitgestellt, so kann ein Laserstrahl von oben her auf dem Probenträger PT entlang der Randkurve R geführt werden, um das Segment S von dem Probenträger PT entlang der Randkurve R abzuschneiden und somit eine Vereinzelung des Segmentes S zu bewirken. Dies ist ebenso für einen Probenträger PTX der Figur 2 möglich.

Befindet sich im Bereich der Randkurve R auf dem Probenträger Blut als Patientenprobe, so wird die optische Energie des Laserstrahls durch den Probenträger PT, PTX besonders gut in dem Fall aufgenommen, dass der Laserstrahl eine Wellenlänge aus einem Bereich von 380 bis 600 nm aufweist. Durch die hier vorgeschlagene Wahl der Wellenlänge des Lasers wird also der Schnittvorgang zur Vereinzelung des Segmentes S besonders effizient durchgeführt.

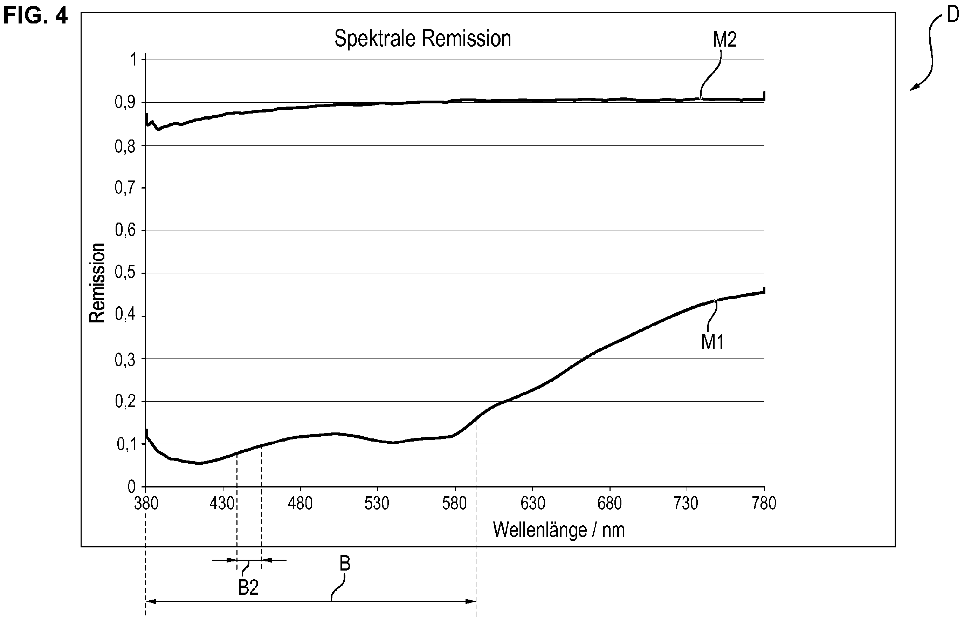

Hierzu zeigt die Figur 4 eine spektrale Remission des Laserlichtes durch einen Probenträger in Abhängigkeit der Wellenlänge des Lasers für zwei unterschiedliche Fälle. Der verwendete Probenträger ist hierbei vom Typ "PerkinElmer 226 Sample Collection Device", welcher als Probenträgermaterial das "Ahlstrom 226 grade paper" aufweist. Die Messkurve M2 illustriert eine spektrale Remission für den Fall, dass der Probenträger im Schnittpunkt des Laserstrahls mit dem Probenträger kein Blut aufweist. Die Messkurve M1 illustriert eine spektrale Remission für den Fall, dass der Probenträger im Schnittpunkt des Laserstrahls mit dem Probenträger Blut aufweist. Der bevorzugte Bereich B von 380 bis 600 nm zur Wahl der Wellenlänge des Lasers ist besonders vorteilhaft, da hier aufgrund der Kombination des Probenträgers auf Baumwollbasis und Blut als Patientenprobe besonders wenig optische Energie von dem Probenträger zurückgestrahlt wird und eben von der Kombination aus Probenträgermaterial auf Baumwollbasis und Blut absorbiert wird, um ein Durchschneiden des Probenträgermaterials zu unterstützen. Insbesondere für die erfindungsgemäße Wahl der Wellenlänge von 440 bis 455 nm, eingetragen als der Bereich B2, ist dieser Effekt auch vorhanden.

Die Figur 3 zeigt eine Ausführungsform eines Probenträgers PTZ, bei welchem der Probenträger PTZ eine bzw. mehrere Aussparungen bzw. Löcher A aufweist, sodass ein Segment S durch einen oder mehrere Stege ST mit dem Rest des Probenträgers PTZ verbunden ist, vorzugsweise lediglich nur noch durch einen oder mehrere Stege ST. Die Aussparungen bzw. Löcher A durchdringen alle Schichten des Probenträgers PTZ. Die Randkurve R eines Segmentes S verläuft somit nicht vollumfänglich auf bzw. in einem Probenträgermaterial des Probenträgers PT, sondern lediglich im Bereich der Stege ST. Daher ist für eine Vereinzelung des Segmentes S ein Durchschneiden des Probenträgers PTZ lediglich im Bereich der Stege ST erforderlich.

Wird auf dem Probenträger PTZ im linken Segment S eine zunächst flüssige Patientenprobe P aufgebracht, so verläuft die Patientenprobe P aufgrund von Kapillarwirkung in dem Segement S und auch hin zu den Stegen ST, so dass eine Überschussmenge an Patientenprobe P, welche nicht im Bereich des Segmentes S von dem Probenträgermaterial aufgenommen werden kann, über die Stege ST hin zu weiteren Bereichen des Probenträgers PTZ verlaufen kann. Hierdurch wird es ermöglicht, dass eine definierte Menge bzw. ein definiertes Volumen an Probenmaterial im Bereich des Segmentes S aufgenommen wird, so dass im Zuge einer späteren biochemischen Analyse lediglich eine definierte Menge bzw. ein bestimmtes Volumen an Probenmaterial prozessiert wird. Dies kann sich positiv auf eine Qualität eines zu erwartenden Analyseergebnisses auswirken.

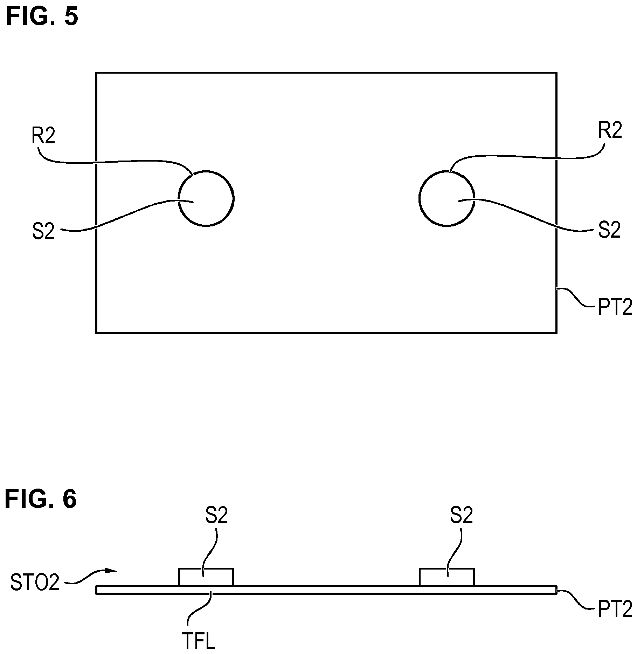

Die Figur 5 zeigt eine weitere bevorzugte Ausführungsform eines Probenträgers PT2, bei welchem die Segmente S2 Material der zuvor erwähnten Stoffschicht aufweisen, wobei jedoch die Stoffschicht STO2 auf Bereiche innerhalb der jeweiligen Randkurven R2 der jeweiligen Segmente S2 begrenzt ist. Die Figur 6 zeigt hierzu eine Seitenansicht des Probenträgers PT2, aus welcher ersichtlich wird, dass lediglich im Bereich der Segmente S2 Material der Stoffschicht STO2 vorhanden ist. Die Segmente S2 befinden sich hierbei auf einer Thermoplastfolie TFL, welche vorzugsweise aus Polyethylen oder Polypropylen besteht. Eine solche Ausgestaltung eines Probenträgers PT2 ist vorteilhaft, da mittels des Laserstrahls im Bereich der Randkurve R2 lediglich die Thermoplastfolie TFL durchtrennt werden muss, nicht aber Material der Stoffschicht STO2, um ein Segment S2 aus dem Probenträger PT2 rauszuschneiden.

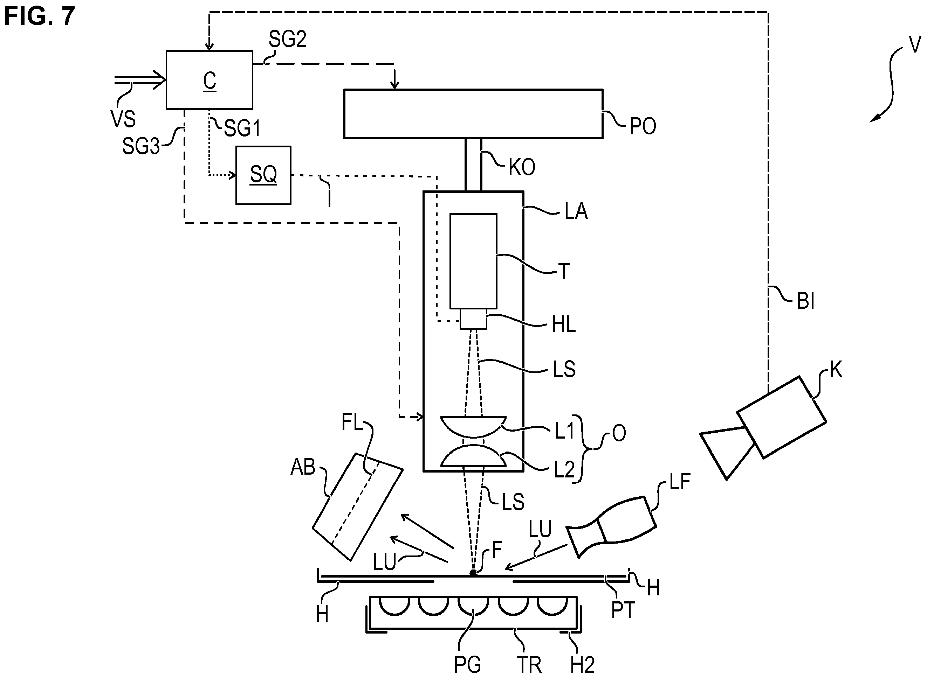

Die Figur 7 zeigt eine bevorzugte Ausführungsform der erfindungsgemäßen Vorrichtung V. Die Vorrichtung weist eine Lasereinheit LA auf, welche wiederum einen Halbleiterlaser HL aufweist. Der Halbleiterlaser HL ist mechanisch und thermische mit einem Thermoelement T zum Abführen von thermischer Energie gekoppelt. Der Halbleiterlaser HL wird von einer Konstantstromquelle SQ mit einem Strom I gespiesen.

Die Lasereinheit LA bzw. der Halbleiterlaser HL emittiert einen Laserstrahl LS, welcher mittels einer Optikeinheit O zu einem Fokuspunkt F hin fokussiert wird, welcher in einer optischen Ebene liegt, die mit einem Probenträger PT zusammenfällt. Der Probenträger PT wird hierbei in einer Halterung H gehalten. Die Optikeinheit O zur Fokussierung des Laserstrahls LS weist wenigstens zwei optische Linsen L1, L2 auf, welche vorzugsweise jeweilige asphärische optische Linsen L1, L2 sind.

Wenigstens ein Positionierungsmittel PO ist mit der Lasereinheit LA verbunden, beispielsweise über eine direkte mechanische Kopplung KO. Die mechanische Kopplung kann als integraler Bestendteil des Positionierungsmittels aufgefasst werden. Das Positionierungsmittel PO bewirkt mittels der mechanischen Kopplung ein Führen der Lasereinheit LA derart, dass der Fokuspunkt F in der optischen Ebene, welche mit dem Probenträger zusammenfällt und eine zweidimensionale Ebene bildet, verbleibt aber innerhalb dieser zweidimensionalen Ebene verschoben wird. Hierdurch wird der Laserstrahl LS bzw. der Fokuspunkt F auf der Ebene des Probenträgers PT geführt.

Das Positionierungsmittel PO ist beispielsweise ein Schrittmotor, dessen Rotationen über eine mechanische Kopplung KO in Form einer Exzenterscheibe auf die Lasereinheit LA übertragen werden, so dass der Fokuspunkt F auf dem Probenträger PT eine Kreisbahn beschreibt. Hierzu steuert eine Steuereinheit C über ein Steuersignal SG2 das Positionierungsmittel entsprechend an. Die Steuereinheit C steuert ferner über ein Steuersignal SG1 die Konstantstromquelle SG an, um eine Zuführung des Stromes I an den Halbleiterlaser HL zu kontrollieren. Das wenigstens eine Positionierungsmittel PO kann ein einzelnes Positionierungsmittel wie z.B. ein Schrittmotor sein, es können aber alternativ auch mehrere Positionierungsmittel vorgesehen sein, um mittels der mechanischen Kopplung ein Führen der Lasereinheit LA derart zu bewirken, dass der Fokuspunkt F auf Höhe der optischen Ebene, welche mit dem Probenträger zusammenfällt und eine zweidimensionale Ebene bildet, verbleibt aber innerhalb dieser zweidimensionalen Ebene verschoben wird.

Nach Ausschneiden eines Segmentes aus dem Probenträger PT fällt das Segment aufgrund der Schwerkraftwirkung in ein Probengefäß PG eines Trägers TR. Vorzugsweise kann die Vorrichtung V Haltemittel H2 zum Halten des Trägers TR aufweisen. Die Haltemittel H2 sind vorzugsweise in der Weise verstellbar, dass der Träger TR mit dem darin befindlichen Probengefäß PG gegenüber dem Probenträger PT ausgerichtet werden kann.

Das Führen des Laserstrahls LS erfolgt also unter Kontrolle des Positionierungsmittels PO mittels Erzeugen einer Relativbewegung zwischen dem Laserstrahl LS und dem Probenträger PT. In der hier dargestellten Ausführungsform bewirkt das Positionierungsmittel eine Änderung einer Position der Lasereinheit LA bzw. des Fokuspunktes F gegenüber dem Probenträger PT. Zusammenfassend kann gesagt werden, dass das Positionierungsmittel eine Position des Laserstrahls in Relation zu dem Probenträger ändert.

Alternativ ist es denkbar, dass ein Positionierungsmittel vorgesehen ist, welches mit der Halterung H des Probenträgers PT gekoppelt ist und welches durch ein hier nicht dargestelltes Steuersignal von der Steuereinheit C angesteuert wird. Hierbei wird unter Kontrolle eines solchen Positionierungsmittels eine Relativbewegung zwischen dem Laserstrahl LS und dem Probenträger PT erzeugt. Es bewirkt dann also das Positionierungsmittel eine Änderung einer Position des Probenträgers PT gegenüber der Lasereinheit LA bzw. des Fokuspunktes F. Auch hier kann zusammenfassend gesagt werden, dass das Positionierungsmittel eine Position des Laserstrahls in Relation zu dem Probenträger ändert.

Eine Luftfördereinheit LF erzeugt einen Luftstrom LU, welcher zumindest teilweise von einerAblufteinheit AB abgesaugt wird. Vorzugsweise weist die Ablufteinheit AB eine Filtereinheit FL zum Reinigen des Luftstromes LU auf.

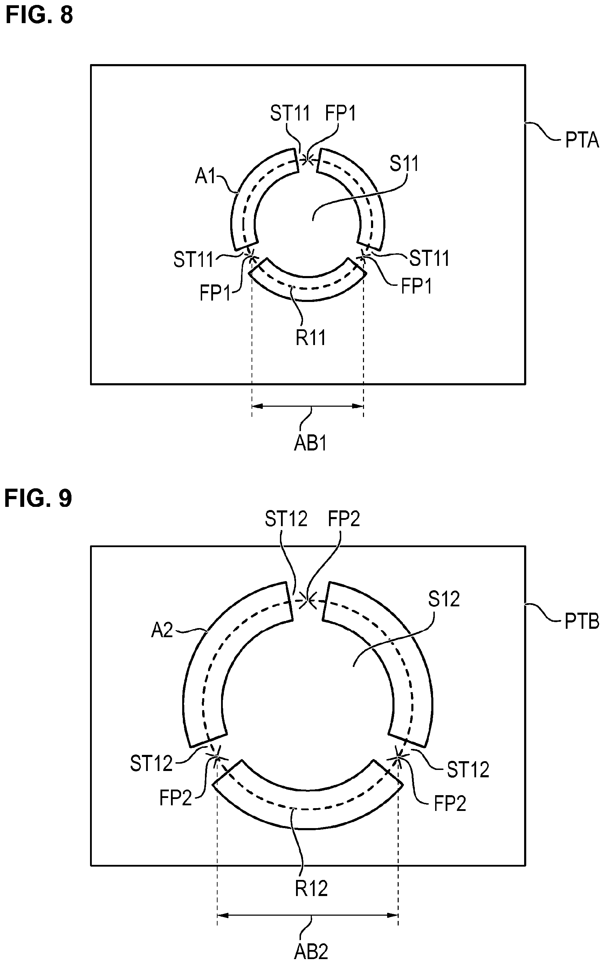

Die Figuren 8 und 9 zeigen unterschiedliche Ausführungsformen eines Probenträgers PTA, PTB bei welchen die jeweiligen Segmente S11, S12 jeweilige unterschiedliche Abmessungen aufweisen. Im Beispiel des Probenträgers PTA der Figur 8 sind wiederum Aussparungen bzw. Löcher A1 vorgesehen, sodass lediglich Stege ST11 verbleiben, entlang welcher ein Durchtrennen des Probenträgers PT mittels eines Laserstrahls erfolgen muss. Anhand von Kreuzen sind jeweilige Fokuspunkte FP1 eingetragen, an welchen jeweilige Laserstrahlen ein jeweiliges Durchschneiden eines jeweiligen Steges ST11 vornehmen können. Die Fokuspunkte FP1 bzw. die dazugehörigen Laserstrahlen müssen dann jeweils im Bereich der entsprechenden Stege ST11 entlang der Randkurve R11 geführt werden, um ein vollständiges Abschneiden des Segmentes S11 von dem Probenträger PTA zu bewirken. Zwei benachbarte Fokuspunkte FP1 haben hierbei einen Abstand AB1 voneinander, welcher beispielsweise 3,5 mm beträgt. Das zuvor beschriebene Positionierungsmittel kann dann beispielsweise ein gleichzeitiges Führen jeweiliger Laserstrahlen jeweiliger Teil-Lasereinheiten so vornehmen, dass die Stege ST11 entlang der Randkurve R11 gleichzeitig durchgeschnitten werden.

Die Figur 9 zeigt eine weitere Ausführungsform eines Probenträgers PTB, welche sich von dem Probenträger PTA der Figur 8 dadurch unterscheidet, dass benachbarte Fokuspunkte FP2 einen Abstand AB2 von beispielsweise 5mm zueinander aufweisen. Die Fokuspunkte FP2 bzw. die dazugehörigen Laserstrahlen müssen dann jeweils im Bereich entsprechender Stege ST2 entlang einer Randkurve R12 geführt werden, um ein vollständiges Abschneiden des Segmentes S12 von dem Probenträger PTB zu bewirken. Auch hier kann das zuvor beschriebene Positionierungsmittel dann beispielsweise ein gleichzeitiges Führen jeweiliger Laserstrahlen jeweiliger Teil-Lasereinheiten so vornehmen, dass die Stege ST12 entlang der Randkurve R12 gleichzeitig durchgeschnitten werden.

Sollen aus unterschiedlichen Probenträgern PTA, PTB jeweils unterschiedlich dimensionierte Segmente S11, S12 in der in Bezug auf die Figuren 8 bzw. 9 beschriebenen Weise ausgeschnitten werden, so kann dies mittels der Vorrichtung V aus der Figur 7 dann erfolgen, wenn die Vorrichtung mehrere Halbleiterlaser bzw. mehrere Teil-Lasereinheiten als Komponenten der Lasereinheit LA aufweist. Hierbei müssen die jeweiligen Fokuspunkte FP1 bzw. FP2 der jeweiligen Teil-Lasereinheiten entsprechend auf den jeweiligen Abstand AB1 bzw. AB2 benachbarter Fokuspunkte FP1 bzw. FP2 eingestellt sein. Ausgehend von der Lasereinheit LA der Figur 7 mit nur einem Halbleiterlaser HL wird nun erläutert, in welcher Weise die Lasereinheit LA erweitert werden kann, um mittels mehrerer Halbleiterlaser bzw. Teil-Lasereinheiten ein Ausschneiden bzw. eine Vereinzelung eines Segmentes aus einem Probenträger unter Berücksichtigung einer variablen Dimensionierung eines Segmentes durchzuführen.

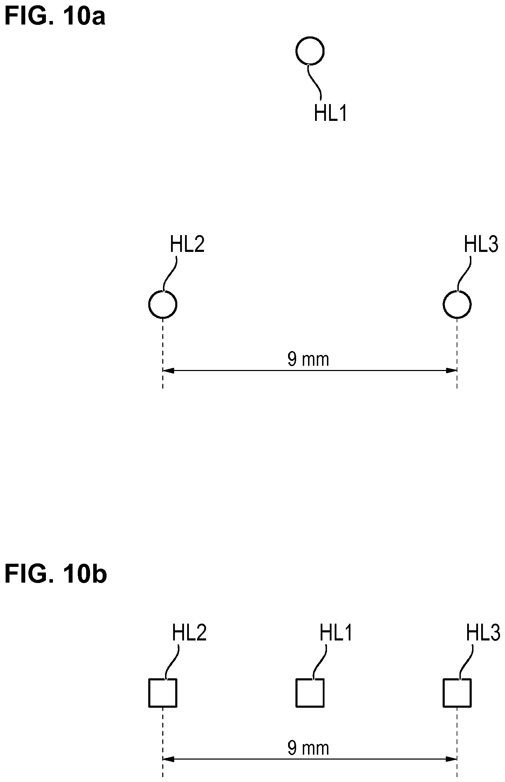

Hierzu zeigt die Figur 10a beispielhaft eine geometrische Anordnung mehrere Halbleiterlaser bzw. Teil-Lasereinheiten HL1, HL2, HL3. Die Figur 10b zeigt hierzu die Anordnung der Teil-Lasereinheiten HL1, HL2, HL3 in einer Seitenansicht. Die Teil-Lasereinheiten HL1, HL2, HL3 sind dann anstelle des Halbleiterlasers HL in der Figur 7 so vorzusehen, dass die Teil-Lasereinheiten HL1, HL2, HL3 in einer Ebene parallel zu der optischen Ebene, welche mit dem Probenträger PT zusammenfällt, angeordnet sind. Benachbarte Teil-Lasereinheiten HL1, HL2, HL3 haben einen Abstand von beispielsweise 9 mm zueinander, welcher sich von einem Abstand AB1 bzw. AB2 zu erzeugender Fokuspunkte FP1 bzw. FP2 aus den Figuren 8 bzw. 9 unterscheidet.

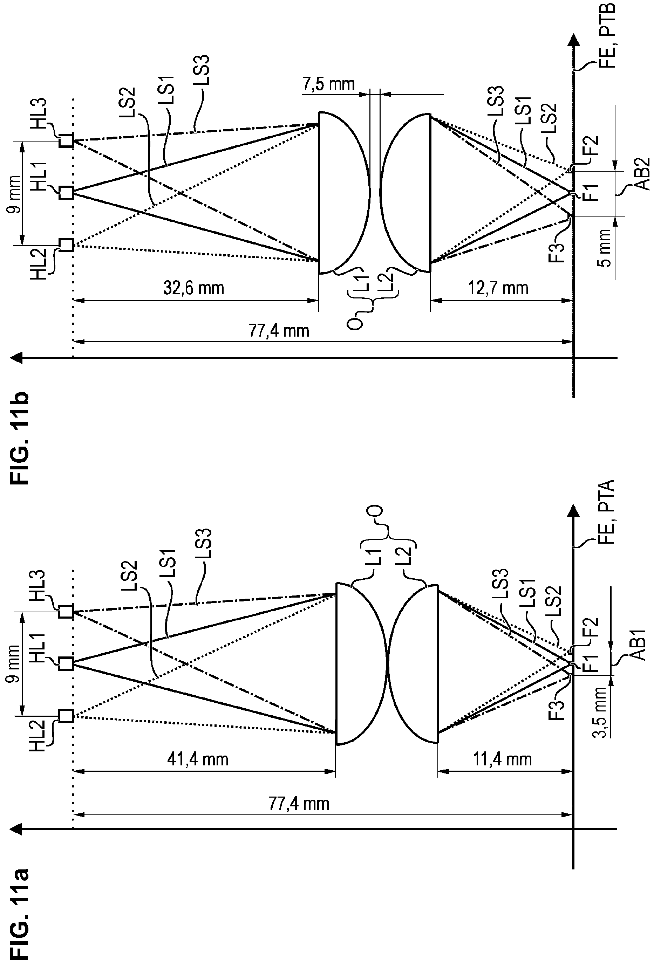

Die Figur 11a zeigt in einer Seitenansicht, in welcher Weise jeweilige Laserstrahlen LS1, LS2, LS3 der jeweiligen Teil-Lasereinheiten HL1, HL2, HL3 durch die optische Einheit O geführt werden können. Die Darstellung in der Figur 11a ist nur beispielhaft und nicht maßstabsgetreu. Es werden hierbei zwei jeweils asphärische Linsen L1, L2 mit einer jeweiligen Breite von 31,5 mm, einer jeweiligen Höhe von 12,3 mm und einem jeweiligen Fokus bzw. einer jeweiligen Brennweite von 27 mm verwendet. Benachbarte Teil-Lasereinheiten HL2, HL3 weisen einen Abstand von 9 mm zueinander auf, wie zuvor unter Bezug auf die Figur 10a erläutert. Die Teil-Lasereinheiten HL1, HL2, HL3 weisen einen Abstand von 77,4 mm zu dem Probenträger PTA bzw. der optischen Fokusebene FE auf. Die erste Linse L1 weist einen Abstand von 41,4 mm zu den Teil-Lasereinheiten HL1, HL2, HL3 auf. Die zweite Linse L2 weist einen Abstand von 11,4 mm zu dem Probenträger PTA bzw. der optischen Fokusebene FE auf. Die Fokuspunkte F1, F2, F3 in der Fokussierungsebene FE bzw. auf dem Probenträger PT liegen so, dass benachbarte Fokuspunkte F3, F2 einen Abstand AB1 von 3,5 mm zueinander aufweisen.

Die Figur 11b zeigt eine gegenüber der Figur 11a geänderte Einstellung der optischen Einheit O. Aufgrund einer Verwendung des Probenträgers PTB mit einer anderen Dimensionierung eines Segmentes S12 bzw. zugehöriger Stege ST2, siehe Figur 9, gegenüber dem Probenträger PTA, siehe Figur 8, ist es notwendig, einen Abstand benachbarter Fokuspunkte F2, F3 in der Fokussierungsebene FE bzw. auf dem Probenträger PTB zu ändern. Hierbei soll eine Fokussierung der Laserstrahlen LS1, LS2, LS3 in der Fokussierungsebene FE bzw. auf dem Probenträger PTB beibehalten werden, um einen Eintrag optischer Energie an den Fokuspunkten F1, F2, F3 auf den Probenträger PTB zu maximieren. Bei der hier vorgeschlagenen Lösung wird es erreicht, einen Abstand der Teil-Lasereinheiten HL1, HL2, HL3 zueinander und ferner auch einen Abstand der Teil-Lasereinheiten HL1, HL2, HL3 zu der Fokussierungsebene FE bzw. dem Probenträger PTB beizubehalten. Mittels eines Einstellens der optischen Einheit O werden die jeweiligen Laserstrahlen LS1, LS2, LS3 der jeweiligen Teil-Lasereinheiten HL1, HL2, HL3 an jeweilige Schnittpunkte mit dem Probenträger PTA, PTB bzw. an jeweilige Fokuspunkte F1, F2, F3 so gelenkt, dass die jeweiligen Schnittpunkte bzw. die jeweiligen Fokuspunkte F1, F2, F3 mit ihrer Fokussierungsebene FE in der optischen Ebene des Probenträgers PTA, PTB verbleiben. Hierbei erfolgt ein Einstellen eines Abstandes der jeweiligen Schnittpunkte bzw. der jeweiligen Fokuspunkte F1, F2, F3 zueinander dadurch, dass wenigstens ein Abstand der optischen Linsen L1, L2 zueinander eingestellt wird. Vorzugsweise wird ein Abstand wenigstens einer der Linsen L1, L2 zu der Fokussierungsebene FE bzw. zu dem Probenträger PTB eingestellt wird.

Die Figur 11b zeigt hierzu eine beispielhafte Lösung, bei welcher die optischen Linsen L1, L2 einen Abstand von 0,52 mm zueinander aufweisen, welcher bei einer Einstellung aus der Figur 11a nur 0 mm beträgt. Die Darstellung in der Figur 11b ist nur beispielhaft und nicht maßstabsgetreu. Ferner weist ein Abstand der Linse L2 von der Fokussierungsebene FE bzw. dem Probenträger PTB einen Wert von 24,4 mm auf. Dieser Abstand weist bei der Einstellung aus der Figur 11a einen Wert von 18,4 mm auf. Auch ein hier nicht explizit Hierdurch wird es bei Beibehalten eines Abstandes der Teil-Lasereinheiten HL1, HL2, HL3 zu der Fokussierungsebene FE bzw. dem Probenträger PT erreicht, dass die Fokuspunkte F1, F2, F3 in der Fokussierungsebene FE nun so ausgestaltet sind, dass benachbarte Fokuspunkte F2, F3 einen Abstand von 5 mm zueinander aufweisen, welches zu der Ausgestaltung des Probenträgers PTB aus der Figur 10b korrespondiert, und dass die Fokuspunkte F1, F2, F3 in der optischen Ebene, welche mit dem Probenträger PT zusammenfällt, verbleiben.

Es wird also durch die hier beschriebene Lösung ermöglicht, dass unterschiedliche Probenträger mit unterschiedlich dimensionierten Segmenten effizient verarbeitet werden können, um die Segmente zu vereinzeln bzw. mittels mehrerer Laserstrahlen aus dem Probenträger herauszuschneiden, ohne ein Bauteil der vorgeschlagenen Vorrichtung austauschen zu müssen und ohne einen Abstand der Teil-Lasereinheiten gegenüber dem Probenträger verändern zu müssen. Es muss lediglich eine Einstellung der optischen Einheit durch Einstellen von Abständen in Bezug auf die Linsen L1, L2 erfolgen. Ein solches Einstellen erfolgt gemäß der Figur 7 vorzugsweise dadurch, dass die Steuereinheit C ein Steuersignal SG3 an die Optikeinheit O bereitstellt. Die Optikeinheit O stellt dann in Abhängigkeit des Steuersignals SG3 eine oder mehrere Positionen der Linsen L1, L2 in der zuvor beschriebenen Weise ein. Hierzu kann die Optikeinheit O jeweilige Positionierungsmittel für die jeweiligen Linsen L1, L2 vorsehen.

Zusammengefasst kann also festgestellt werden, dass durch die Optikeinheit O die jeweiligen Laserstrahlen LS1, LS2, LS3 an die jeweiligen, unterschiedlichen Schnittpunkte auf der Randkurve R11, R12 derart gelenkt werden, dass sich für die jeweiligen Laserstrahlen LS1, LS2, LS3 in einer optischen Ebene, welche mit dem Probenträger PTA, PTB zusammenfällt, jeweilige Fokuspunkte FP1 ergeben, und dass die Optikeinheit derart einstellbar ist, dass sich für einen veränderten bzw. unterschiedlichen Abstand der jeweiligen Schnittpunkte der jeweiligen Laserstrahlen LS1, LS2, LS3 in der optischen Ebene auf dem Probenträger PTA, PTB zueinander in der optischen Ebene auf dem Probenträger PTA, PTB entsprechende, jeweilige Fokuspunkte FP1 ergeben.

Die Vorrichtung V aus der Figur 7 ist vorzugsweise derart ausgestaltet, dass die Steuereinheit C ein Vorgabesignal VS entgegennehmen kann, welches eine Segmentgröße S11, S12 der Segmente aus den Figuren 8 bzw. 9 oder aber einen gewünschten Abstand AB1, AB2 benachbarter Fokuspunkte bzw. Schnittpunkte auf dem Probenträger direkt oder indirekt indiziert. Beispielsweise kann dieses Vorgabesignal VS als ein Eingabesignal durch einen Nutzer in eine Nutzerschnittstelle eingegeben und an die Steuereinheit C bereitgestellt werden. Alternativ kann die Steuereinheit C ein solches Vorgabesignal VS über eine Datenschnittstelle entgegennehmen. Alternativ kann die Vorrichtung V eine Bilderfassungseinheit K in Form einer Kamera aufweisen, welche eine Bildinformation BI bereitstellt, welche jenen Teilbereich des Probenträgers repräsentiert, in welchem sich das zu vereinzelnde Segment befindet. Die Steuereinheit C kann dann diese Bildinformation BI auswerten und eine Segmentgröße oder einen Abstand benachbarter Schnittpunkte bzw. Fokuspunkte selber bestimmen.

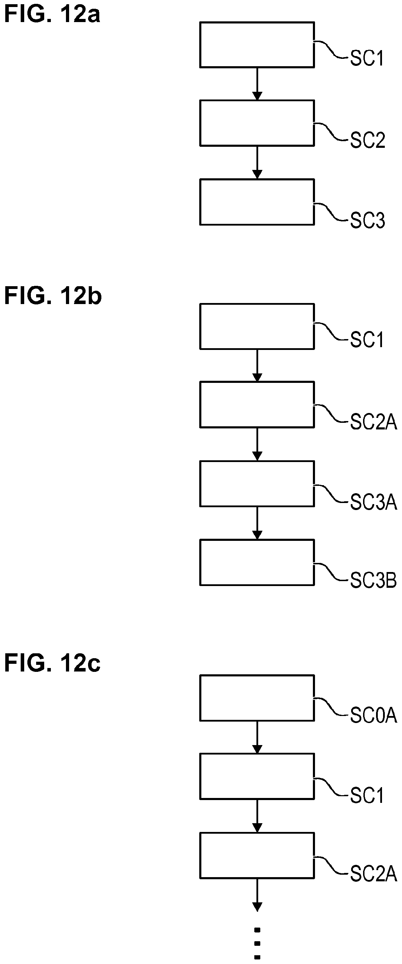

Die Figur 12a Art zeigt Verfahrensschritte SC1, SC2, SC3, welche im Zuge des erfindungsgemäßen Verfahrens durchgeführt werden. In dem Verfahrensschritt SC1 wird ein Probenträger, welcher geeignet ist, eine flüssige Patientenprobe aufzunehmen, bereitgestellt, wobei der Probenträger in oder auf einem Segment zumindest einen Teil der Patientenprobe aufweist.

In dem Verfahrensschritt SC2 wird mittels einer Lasereinheit ein Laserstrahl erzeugt. In dem Verfahrensschritt SC3 wird mittels Führung des Laserstrahls entlang zumindest eines Teils einer Randkurve des Segmentes das Segment von dem Probenträger abgeschnitten. Hierbei weist der Laserstrahl eine Wellenlänge in einem Bereich von 380 bis 600 nm auf.

Gemäß des Beispiels aus der Figur 12b kann nach dem Bereitstellen des Probenträgers im Zuge des Schrittes SC1 anschließend in einem Verfahrensschritt SC2A, welcher alternativ zu dem Verfahrensschritt SC2 aus der Figur 12a vorgenommen werden kann, es vorgesehen sein, mittels jeweiliger Teil Lasereinheiten mehrere, jeweilige Laserstrahlen zu erzeugen.

Auf den Verfahrensschritt SC2A folgen dann Verfahrensschritte SC3A und SC3B, welche alternativ zu dem Verfahrensschritt SC3 der Figur 12a durchgeführt werden können. In dem Verfahrensschritt SC3A erfolgt ein Lenken der jeweiligen Laserstrahlen an jeweilige, unterschiedliche Schnittpunkte auf der Randkurve mittels einer Optikeinheit. In dem Verfahrensschritt SC3B erfolgt ein Abschneiden des Segmentes von dem Probenträger mittels gleichzeitigen Führens der jeweiligen Laserstrahlen entlang jeweiliger Teilabschnitte der Randkurve des Segmentes.

Gemäß der Figur 12c kann vor Durchführung des Verfahrensschrittes SC1 aus der Figur 12b ein zusätzlicher Verfahrensschritt SCOA vorgesehen sein. Im Zuges des Schrittes SCOA wird ein Abstand der jeweiligen Schnittpunkte der jeweiligen Laserstrahlen auf dem Probenträger dadurch eingestellt wird, dass die Optikeinheit eingestellt wird. Dieses erfolgt vorzugsweise durch Einstellen eines Abstandes einer ersten optischen Linse zu einer zweiten optischen Linse sowie Einstellen eines Abstandes der ersten optischen Linse zu den Teil Lasereinheiten.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung die Steuereinheit in Hardware und/oder in Software umsetzen. Eine Umsetzung der hier genannten Steuereinheit kann hier als wenigstens eine Steuereinheit erfolgen oder aber durch mehrere Steuereinheiten im Verbund. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann als Steuereinheit durch einen Prozessor, einen Computerprozessor (CPU = Central Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird

Allgemein können Ausführungsbeispiele oder Teile der Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren oder ein Teil eines Verfahrens durchzuführen, wenn das Programm auf einem Prozessor oder einer programmierbaren Hardwarekomponente abläuft.

Im Folgenden werden Beispiele zur Segmentierung bzw. Vereinzelung von Patientenproben bzw. Segmenten mit Patientenproben angegeben.

**Beispiel 1: Laserseparation**

**Verwendete ELISA Assays (Hersteller: EUROIMMUN)**

| Material | Bestellnummer |
|---|---|
| Anti-Röteln-Viren-IgG-ELISA | EI 2590-9601 G |
| 25-OH-Vitamin D-ELISA | EQ 6411-9601 |

**Verwendete Geräte**

Washer HydroFlex (Tecan); Stanzzange (McGill), Durchmesser 4,75mm; Stanzvorrichtung zum Vorstanzen von Stegen (nicht kommerziell vertriebener Eigenbau); Laservorrichtung (nicht kommerziell vertriebener Eigenbau); Photometer Sunrise (Tecan), Magellan-Software

Die Laservorrichtung als nicht kommerziell vertriebener Eigenbau wies eine einzelne Lasereinheit bzw. einen einzelnen Halbleiterlaser des Typs NLD453500G des Herstellers "Roithner Lasertechnik" mit einer optischen Ausgangsleistung von 3,5 Watt auf. Die optische Einheit bestand aus zwei asphärischen Linsen mit einem einer Brennweite von f=27 mm. Der Halbleiterlaser wurde mit einem Laserstrom von I=2,6 Ampere angesteuert. Als Positioniereinheit wurde eine Motor mit Drehachse verwendet, welche über eine Exzenterscheibe als Koppeleinheit mit der Lasereinheit mechanisch gekoppelt war, so dass die Motordrehung der Drehachse eine Positionierung der Lasereinheit derart bewirkte, dass der Laserstrahl bzw. dessen Fokuspunkt auf dem Probenträger eine kreisförmige Umlaufkurve bzw. Randkurve eines Durchmessers von ungefähr 4,75 mm beschrieb. Die Drehfrequenz betrug hierbei 1/6 Herz, so dass eine vollumfängliche Randkurve eines Segmentes durch den Laserstrahl der einzelnen Lasereinheit in 6 Sekunden abgefahren wurde.

Bei dem verwendeten Probenträger handelte es sich um das Produkt "PerkinElmer 226 Sample Collection Device" mit dem Probenträgermaterial "Ahlstrom 226 grade paper"

**Durchführung**

Segmente mit getrockneten Blutstropfen (DBS) in definierter Größe wurden auf drei verschiedene Arten aus Probenträgern ausgeschnitten bzw. vereinzelt. Die kreisförmigen Segmente wiesen eine Größe von ungefähr 4,75 mm auf.

Die erste Art (1.) bestand darin, nach Aufbringen der Patientenprobe die Segmente mittels einer Stanzzange (McGill) aus dem Probenträger herauszustanzen. Die zweite Art (2.) bestand darin, die Segmente nach Aufbringen der Patientenprobe mittels der zuvor beschriebenen Laservorrichtung vollumfänglich entlang einer kreisförmigen Randkurve auszuschneiden. Die dritte Art (3.) bestand darin, Probenträger vor Aufbringen der Patientenprobe mittels einer selbst gebauten Stanzvorrichtung derart vorzustanzen, dass ein Segment nur noch durch drei Stege mit dem Rest des Probenträgers verbunden waren, wobei jeder Steg eine Breite von ca. 1,6 mm aufwies, und nach Aufbringen der Patientenprobe anschließend die Segmente mittels der zuvor beschriebenen Laservorrichtung entlang der Stege auszuschneiden.

Die ausgeschnittenen Segmente wurden als Proben in ELISA Assays verwendet und hierdurch jeweilige Ergebnisse für die jeweiligen Arten des Ausschneidens bzw. der Vereinzelung erhalten. Die jeweiligen Ergebnisse wurden miteinander verglichen. Schneiden mit dem Laserstrahl kann hohe Temperaturen erzeugen. Zur Beurteilung, ob diese hohen Temperaturen einen Einfluss auf den Analyt und dessen weitere Verwendung, insbesondere in ELISA Assays, haben, wurden Probeninkubationen mit Blut von fünf verschiedenen Patienten durchgeführt.

Zur Herstellung der DBS wurde PerkinElmer 226 Probensammelpapier verwendet. Das Papier wurde im Original eingesetzt bzw. wie zuvor beschreiben mit einer Stanzvorrichtung vorgestanzt. Das Blut wurde auf das Papier getropft und getrocknet und anschließend per Laser oder durch Stanzen ausgeschnitten. Die DBS wurden wie zuvor beschrieben

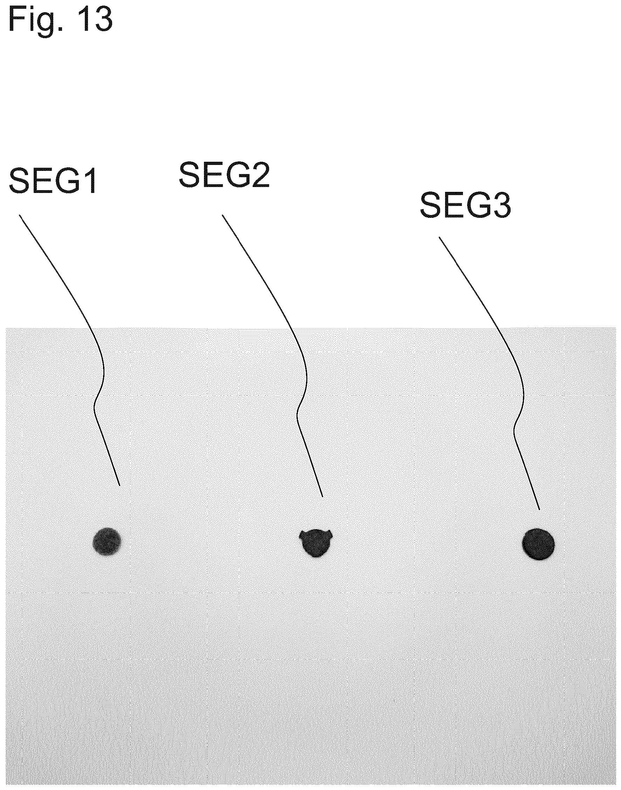

1. mit einer Zange aus dem betropften Papier ausgestanzt (Kurzbezeichnung: Zange) = Referenzproben, in der Figur 13 beispielhaft als SEG1 gezeigt,

2. mit einem Laser aus einem vorgestanzten, betropften Papier geschnitten (Kurzbezeichnung: Stege), in der Figur 13 beispielhaft als SEG2 gezeigt, und

3. mit einem Laser aus dem betropften Papier komplett durch Laserseparation geschnitten, in der Figur 13 beispielhaft als SEG3 gezeigt.

Die mit der Zange gestanzten DBS (1.) dienten bei der Inkubation als Referenzproben, da diese Methode zuvor validiert wurde. Die Größe der gestanzten DBS ist vergleichbar mit den lasergeschnittenen DBS, aber im Gegensatz zur Laserseparation tritt bei der Zangenbehandlung keine Hitzeentwicklung auf.

Zur Analyse wurden die verschieden generierten DBSmit einem Antigen- (25-OH-Vitamin D-ELISA) und einem Antikörpernachweis (Anti-Röteln-Viren-IgG ELISA) untersucht.

Tabelle 1: Übersicht der verwendeten Proben

| Patient Nr. | Anti-Röteln-Viren-IgG-ELISA | 25-OH-Vitamin D-ELISA |
|---|---|---|
| 1 | Zange<br>Stege<br>Kreise | Zange<br>Stege |
| 2 | Zange<br>Stege<br>Kreise | Zange<br>Stege |
| 3 | Zange<br>Stege<br>Kreise | Zange<br>Stege |
| 4 | Zange<br>Stege<br>Kreise | Zange<br>Stege |
| 5 | Stege<br>Kreise | Zange<br>Stege |

## Anti-Röteln-IgG ELISA

Für den Anti-Röteln-IgG ELISA wurden von jedem Patienten drei DBS untersucht, die jeweils durch die oben beschriebenen verschiedenen Verfahren erzeugt wurden. Nach dem Stanzen oder Lasern wurde je ein DBS in ein Probengefäß gegeben, mit 250 μl Probenpuffer versetzt und für eine Stunde bei 37°C extrahiert. Anschließend wurden 100 μl dieses Extrakts als Probenverdünnung in eine Vertiefung der beschichteten Mikrotiterplatte überführt. Die Probeninkubation erfolgte 30 Minuten bei Raumtempratur. Anschließend wurde 3x mit je 450 μl gebrauchsfertigen Waschpuffer gewaschen. Dann wurden jeweils 100 μl Enzymkonjugat (Peroxidase-markiertes Anti-Human-IgG) in die Reagenzgefäße pipettiert und 30 Minuten bei Raumtemperatur inkubiert. Es folgte ein Waschschritt wie zuvor (3x 450 μl s.o.). Es wurden 100 μl Substrat-Lösung in die Reagenzgefäße pipettiert und für 15 Minuten inkubiert. Anschließend wurden 100 μl Stopp-Lösung dazugegeben und photometrisch ausgewertet. Die Ermittlung der relativen Einheiten der Anti-Röteln-Viren-IgG-Antikörper erfolgte anhand der mit inkubierten Kalibratoren.

## Ergebnisse zu Anti-Röteln-IgG ELISA

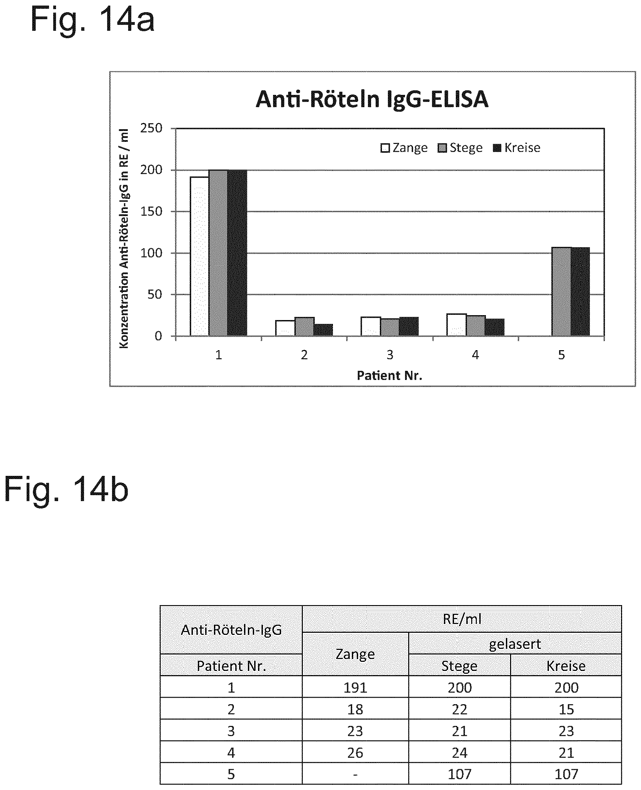

Eine tabellarische Auswertung der Ergebnisse findet sich in Figur 14b. Es wurden für Patient Nr. 5 keine Segmente mittels der Stanzzange ausgeschnitten. Eine graphische Auswertung der Ergebnisse findet sich in Figur 14a.
Im Antikörpernachweis (Anti-Röteln-IgG ELISA) erhält man vergleichbare Ergebnisse mit den unterschiedlich gestanzten oder mittels Laser geschnittenen DBS. Die Proben werden durch den Laser nicht zerstört oder beeinträchtigt.

## 25-OH-Vitamin D-ELISA

Für den 25-OH-Vitamin D-ELISA wurden von jedem Patienten zwei verschiedene DBS untersucht, die durch Ausstanzen mittels der Stanzzange oder Laserschneiden mittels des Laserstrahls von bereits zur Erzeugung von Stegen vorgestanztem, betropftem Papier erzeugt wurden. Zur Untersuchung der Proben wurde zunächst Vitamin D-Biotin 1:100 mit Harnstoff-Probenpuffer verdünnt. Anschließend wurden die DBS in ein Probengefäß gegeben, mit 250 μl gebrauchsfertigem Biotin versetzt und für eine Stunde bei Raumtemperatur auf einem Schüttler extrahiert. Anschließend wurden 100 μl dieses Extrakts als Probenverdünnung in eine Vertiefung der beschichteten Mikrotiterplatte überführt. Die Probeninkubation erfolgte 2 Stunden bei Raumtempratur. Anschließend wurde 3x mit je 450 μl gebrauchsfertigen Waschpuffer gewaschen. Dann wurden jeweils 100 μl Enzymkonjugat (Peroxidase-markiertes Streptavidin) in die Reagenzgefäße pipettiert und 30 Minuten bei Raumtemperatur inkubiert. Es folgte ein Waschschritt wie zuvor (3x 450 μl s.o.). Es wurden 100 μl Substrat-Lösung in die Reagenzgefäße pipettiert und für 15 Minuten inkubiert. Anschließend wurden 100 μl Stopp-Lösung dazugegeben und photometrisch ausgewertet. Die Ermittlung der 25-OH-Vitamin D-Konzentrationen erfolgte anhand der mit inkubierten Kalibratoren.

**Ergebnisse zu 25-OH-Vitamin D-ELISA**

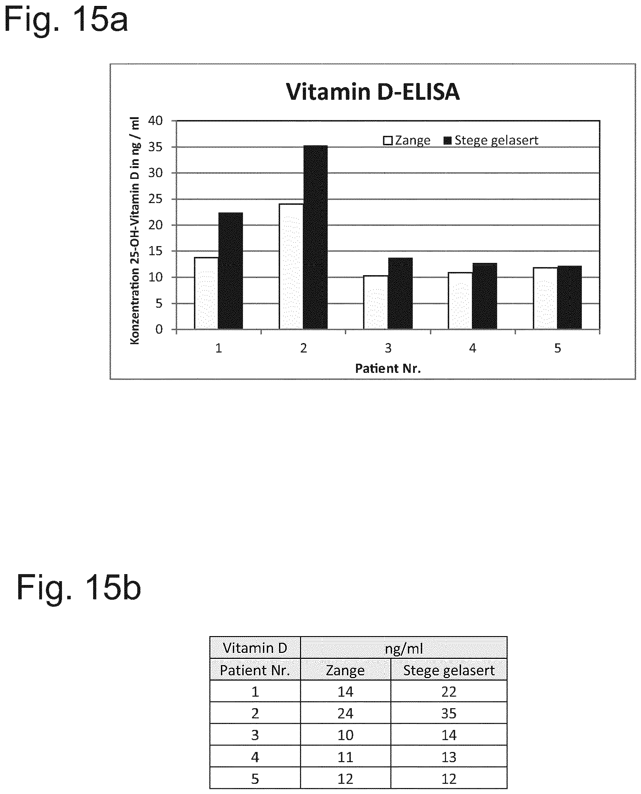

Eine tabellarische Auswertung der Ergebnisse findet sich in Figur 15b. Eine graphische Auswertung der Ergebnisse findet sich in Figur 15a.

Im Antigennachweis (25-OH-Vitamin D-ELISA) erhält man vergleichbare Ergebnisse mit den unterschiedlich gestanzten oder geschnittenen DBS. Die Proben werden durch den Laser nicht zerstört oder beeinträchtigt.

Bezugszeichenliste

| | |
|---|---|
| A | Aussparung |
| AB | Ablufteinheit |
| AB1, AB2 | Abstand |
| B, B2 | Bereich |
| BI | Bildinformation |
| C | Steuereinheit |
| F, FP1, FP2, F1, F2, F3 | Fokuspunkt |
| FE | Fokussierungsebene |
| FL | Folienschicht |
| FL | Filtereinheit |
| H | Halterung |
| H2 | Haltemittel |
| HL | Halbleiterlaser |
| HL1, HL2, HL3 | Teil-Lasereinheit |
| I | Strom |
| K | Kamera |
| KO | Kopplung |
| LA | Lasereinheit |
| L1 | Linse 1 |
| L2 | Linse 2 |
| LF | Luftfördereinheit |
| LS, LS1, LS2, LS3 | Laserstrahl |
| LU | Luftstrom |
| M1, M2 | Messkurve |
| O | Optikeinheit |
| P | Patientenprobe |
| PG | Probengefäß |
| PO | Positionierungsmittel |
| PT, PT2 PTX, PTZ, PTA, PTB | Probenträger |
| R, R2, R11, R12 | Randkurve |
| S, S2, S11, S12 | Segment |
| SC1, SC2, SC3, SC2A, SC3A, SC3B, SCOA SEG1, SEG2, SEG3 | Verfahrensschritte Probensegmente |
| SG1, SG2, SG3 | Steuersignal |
| SQ | Konstantstromquelle |
| ST, ST11, ST12 | Steg |
| STO, STO2 | Stoffschicht |
| T | Thermoelement |
| TFL | Thermoplastfolie |
| TR | Träger |
| V | Vorrichtung |
| VS | Vorgabesignal |

EP 3 567 362 B1

**Patentansprüche**

1. Verfahren zur Segmentierung und Vereinzelung flächig verteilter Patientenproben, wobei ein Probenträger (PT, PT2, PTX, PTZ, PTA, PTB) geeignet ist, eine flüssige Patientenprobe (P) aufzunehmen, und wobei der Probenträger eine Stoffschicht aufweist, die geeignet ist, die flüssige Patientenprobe zu adsorbieren, aufweisend die Schritte

   - Bereitstellen des Probenträgers, welcher in oder auf einem Segment (S, S2, S11, S12) zumindest einen Teil der Patientenprobe (P) aufweist,
   - Erzeugen eines Laserstrahls (LS, LS1, LS2, LS3) mittels einer Lasereinheit (LA, HL1, HL2, HL3),
   - Abschneiden des Segmentes (S, S2, S11, S12) von dem Probenträger (PT, PT2, PTX, PTZ, PTA, PTB) mittels Führen des Laserstrahls (LS, LS1, LS2, LS3) entlang zumindest eines Teils einer Randkurve des Segmentes,

   **dadurch gekennzeichnet, dass** die Lasereinheit (LA, HL1, HL2, HL3) ein Halbleiterlaser (HL) ist, welcher eine optische Mindestleistung von mehr als 1 Watt erzeugt, und dass der Laserstrahl (LS, LS1, LS2, LS3) eine Wellenlänge in einem Bereich (B) von 440 bis 455 nm aufweist.

2. Verfahren nach Anspruch 1,
   **dadurch gekennzeichnet, dass** der Probenträger (PTZ, PTA, PTB) Aussparungen aufweist, sodass das Segment (S, S11, S12) mit dem Rest des Probenträgers (PTZ, PTA, PTB) durch einen oder mehrere Stege (ST, ST11, ST12) verbunden ist, ferner **gekennzeichnet durch** Führen des Laserstrahls (LS, LS1, LS2, LS3) entlang jener Teilbereiche der Randkurve (R, R2, R11, R12) des Segmentes (S, S11, S12), an welchen sich die Stege (ST, ST11, ST12) befinden.

3. Verfahren nach Anspruch 1,
   **dadurch gekennzeichnet,**

   - **dass** der Probenträger (PT2) eine flächig durchgängige Folienschicht (TFL) mit einer Thermoplastfolie aufweist,

   und **dass** das Segment (S2) aus einem Stoffschichtsegment (STO2) gebildet wird, welches auf der Thermoplastfolie aufgebracht sind.

4. Verfahren nach Anspruch 1,
   ferner **gekennzeichnet durch**

   - Zuführen eines Luftstromes (LU) hin zu einer Schnittstelle des Laserstrahls (LS, LS1, LS2, LS3) mit dem Probenträger (PT, PT2, PTX, PTZ, PTA, PTB),
   - Absaugen zumindest eines Teils des Luftstromes (LU) von der Schnittstelle weg.

5. Verfahren nach Anspruch 1,
   **gekennzeichnet durch**

   - Erzeugen mehrerer, jeweiliger Laserstrahlen (LS1, LS2, LS3) mittels jeweiliger Teil-Lasereinheiten (HL1, HL2, HL3),
   - Lenken der jeweiligen Laserstrahlen (LS2, LS2, LS3) an jeweilige, unterschiedliche Schnittpunkte auf der Randkurve (R11, R12) mittels einer Optikeinheit (O),
   - Abschneiden des Segmentes (S11, S12) von dem Probenträger (PTA, PTB) mittels gleichzeitigen Führens der jeweiligen Laserstrahlen (LS1, LS2, LS3) entlang jeweiliger Teilabschnitte der Randkurve (R11, R12) des Segmentes (S11, S12).

6. Verfahren nach Anspruch 5,
   ferner **gekennzeichnet durch**

   - Lenken, mittels der Optikeinheit, der jeweiligen Laserstrahlen (LS1, LS2, LS3) an die jeweiligen, unterschiedlichen Schnittpunkte auf der Randkurve (R11, R12) derart, dass sich für die jeweiligen Laserstrahlen (LS1, LS2, LS3) in einer optischen Ebene, welche mit dem Probenträger (PTA, PTB) zusammenfällt, jeweilige Fokuspunkte (FP1) ergeben,
   - und Einstellen der Optikeinheit derart, dass sich für einen veränderten bzw. unterschiedlichen Abstand der jeweiligen Schnittpunkte der jeweiligen Laserstrahlen (LS1, LS2, LS3) in der optischen Ebene auf dem Probenträger (PTA, PTB) zueinander in der optischen Ebene auf dem Probenträger (PTA, PTB) entsprechende, jeweilige Fokuspunkte (FP1) ergeben.

7. Vorrichtung zur Segmentierung und Vereinzelung flächig verteilter Patientenproben (P) mittels Laserschneidens, mit einem Probenträger (PT, PT2, PTX, PTZ, PTA, PTB) der geeignet ist, eine flüssige Patientenprobe (P) aufzunehmen, und wobei der Probenträger eine Stoffschicht aufweist, die geeignet ist, die flüssige Patientenprobe zu adsorbieren,
aufweisend

- eine Halterung (H) zum Halten des Probenträgers (PT, PT2, PTX, PTZ, PTA, PTB), welcher in oder auf einem Segment (S, S2, S11, S12) des Probenträgers (PT, PT2, PTX, PTZ, PTA, PTB), zumindest einen Teil der Patientenprobe (P) aufweist,
- eine Lasereinheit (LA, HL1, HL2, HL3) zum Erzeugen eines Laserstrahls (LS, LS1, LS2, LS3),
- wenigstens ein Positionierungsmittel (PO) zum Ändern einer Position des Laserstrahls (LS, LS1, LS2, LS3) in Relation zu dem Probenträger (PT, PT2, PTX, PTZ, PTA, PTB),
- wenigstens eine Steuereinheit (C) zum Ansteuern des Positionierungsmittels (PO) derart, dass der Laserstrahl (LS, LS1, LS2, LS3) entlang zumindest eines Teils einer Randkurve (R, R2, R11, R12) des Segmentes (S, S2, S11, S12) geführt wird, sodass das Segment (S, S2, S11, S12) von dem Probenträger (PT, PT2, PTX, PTZ, PTA, PTB) abgeschnitten wird,

**dadurch gekennzeichnet, dass** die Lasereinheit (LA, HL1, HL2, HL3) ein Halbleiterlaser (HL) ist, welcher eine optische Mindestleistung von mehr als 1 Watt erzeugt, und dass der Laserstrahl (LS, LS1, LS2, LS3) eine Wellenlänge in einem Bereich von 440 bis 455 nm aufweist.

8. Vorrichtung nach Anspruch 7,

wobei der Probenträger (PTZ, PTA, PTB) Aussparungen aufweist, sodass das Segment (S, S11, S12) mit dem Rest des Probenträgers (PTZ, PTA, PTB) durch einen oder mehrere Stege (ST, ST11, ST12) verbunden ist,
**dadurch gekennzeichnet, dass** die Steuereinheit (C) ausgebildet ist, das Positionierungsmittel (PO) derart anzusteuern, dass der Laserstrahl (LS, LS1, LS2, LS3) entlang jener Teilbereiche der Randkurve (R, R2, R11, R12) des Segmentes (S, S11, S12) geführt wird, an welchen sich die Stege (ST, ST11, ST12) befinden.

9. Vorrichtung nach Anspruch 7,
ferner **gekennzeichnet durch**

- eine Luftfördereinheit (LF) zum Zuführen eines Luftstromes (LU) hin zu einer Schnittstelle des Laserstrahls (LS, LS1, LS2, LS3) mit dem Probenträger (PT, PT2, PTX, PTZ, PTA, PTB),
- sowie eine Ablufteinheit (AB) zum Absaugen zumindest eines Teils des Luftstromes (LU) von der Schnittstelle weg.

10. Vorrichtung nach Anspruch 7,
ferner **gekennzeichnet durch**

- mehrere, jeweilige Teil-Lasereinheiten (HL1, HL2, HL3) zum Erzeugen jeweiliger Laserstrahlen (LS1, LS2, LS3),
- sowie eine Optikeinheit (O) zum Lenken der jeweiligen Laserstrahlen (LS1, LS2, LS3) an jeweilige Schnittpunkte auf der Randkurve (R11, R12),

ferner **dadurch** gekennzeichnet,

- dass das Positionierungsmittel (PO) geeignet ist, jeweilige Positionen der jeweiligen Laserstrahlen (LS1, LS2, LS3) in Relation zu dem Probenträger (PTA, PTB) zu ändern,
- und dass die Steuereinheit (C) ausgebildet ist, das Positionierungsmittel (PO) derart anzusteuern, dass die jeweiligen Laserstrahlen (LS1, LS2, LS3) gleichzeitig entlang jeweiliger Teilabschnitte der Randkurve (R11, R12) geführt werden, sodass das Segment (S11, S12) von dem Probenträger (PTA, PTB) abgeschnitten wird.

11. Vorrichtung nach Anspruch 10,

wobei die Optikeinheit (O) ausgebildet ist, die jeweiligen Laserstrahlen (LS1, LS2, LS3) an die jeweiligen, unterschiedlichen Schnittpunkte auf der Randkurve (R11, R12) so zu lenken, dass sich für die jeweiligen Laserstrahlen (LS1, LS2, LS3) in einer optischen Ebene, welche mit dem Probenträger (PTA, PTB) zusammenfällt, jeweilige Fokuspunkte (FP1) ergeben,

und dass die Optikeinheit so einstellbar ist, dass ein Abstand der jeweiligen Schnittpunkte der jeweiligen Laserstrahlen (LS1, LS2, LS3) in der optischen Ebene auf dem Probenträger (PTA, PTB) zueinander so eingestellt werden kann, dass sich auch für unterschiedliche Abstände (AB1, AB2) der jeweiligen Schnittpunkte in der optischen Ebene auf dem Probenträger (PTA, PTB) entsprechende, jeweilige Fokuspunkte (FP2) ergeben,

wobei insbesondere die Steuereinheit (C) ausgebildet ist, die Optikeinheit (O) mittels eines Steuersignals zur Veränderung bzw. Einstellung des Abstandes der jeweiligen Schnittpunkte in der optischen Ebene auf dem Probenträger (PTA, PTB) anzusteuern.

**12.** System aufweisend

- eine Vorrichtung (V) nach einem der Ansprüche 7 bis 11,
- wobei der Probenträger (PTZ, PTA, PTB) ferner Aussparungen (A) aufweist, sodass eine Segment (S, S11, S12) des Probenträgers mit dem Rest des Probenträgers (PTZ, PTA, PTB) durch einen oder mehrere Stege (ST, ST11, ST12) verbunden ist.

**Claims**

**1.** Method for segmenting and separating patient samples distributed over an area, wherein a sample carrier (PT, PT2, PTX, PTZ, PTA, PTB) is suitable for receiving a liquid patient sample (P) and wherein the sample carrier has a material layer suitable for adsorbing the liquid patient sample, including the steps of

- providing the sample carrier which has at least some of the patient sample (P) in or on a segment (S, S2, S11, S12),
- producing a laser beam (LS, LS1, LS2, LS3) by means of a laser unit (LA, HL1, HL2, HL3),
- cutting the segment (S, S2, S11, S12) from the sample carrier (PT, PT2, PTX, PTZ, PTA, PTB) by guiding the laser beam (LS, LS1, LS2, LS3) along at least part of a boundary curve of the segment,

**characterized in that** the laser unit (LA, HL1, HL2, HL3) is a semiconductor laser (HL) that produces a minimum optical power of more than 1 watt and **in that** the laser beam (LS, LS1, LS2, LS3) has a wavelength in a range (B) from 440 to 455 nm.

**2.** Method according to Claim 1,

**characterized in that** the sample carrier (PTZ, PTA, PTB) has cut-outs such that the segment (S, S11, S12) is connected to the remainder of the sample carrier (PTZ, PTA, PTB) by one or more webs (ST, ST11, ST12), further **characterized by** guiding the laser beam (LS, LS1, LS2, LS3) along those portions of the boundary curve (R, R2, R11, R12) of the segment (S, S11, S12) where the webs (ST, ST11, ST12) are situated.

**3.** Method according to Claim 1,
**characterized in that**

- the sample carrier (PT2) has a two-dimensionally continuous film layer (TFL) with a thermoplastic film,

and **in that** the segment (S2) is formed from a material layer segment (STO2) which is applied to the thermoplastic film.

**4.** Method according to Claim 1,
further **characterized by**

- feeding an airflow (LU) to an interface between the laser beam (LS, LS1, LS2, LS3) and the sample carrier (PT, PT2, PTX, PTZ, PTA, PTB),
- aspirating at least some of the airflow (LU) away from the interface.

**5.** Method according to Claim 1,

**characterized by**

- producing a plurality of respective laser beams (LS1, LS2, LS3) by means of respective laser subunits (HR1, HL2, HL3),
- steering the respective lasers beams (LS1, LS2, LS3) to respective, different cutting points on the boundary curve (R11, R12) by means of an optical unit (O),
- cutting the segment (S11, S12) from the sample carrier (PTA, PTB) by simultaneously guiding the respective laser beams (LS1, LS2, LS3) along respective portions of the boundary curve (R11, R12) of the segment (S11, S12).

6. Method according to Claim 5,
   further **characterized by**

   - using the optical unit to steer the respective laser beams (LS1, LS2, LS3) to the respective, different cutting points on the boundary curve (R11, R12) in such a way that respective focal points (FP1) arise for the respective laser beams (LS1, LS2, LS3) in an optical plane that coincides with the sample carrier (PTA, PTB),
   - and setting the optical unit in such a way that, for an altered or different distance of the respective cutting points of the respective laser beams (LS1, LS2, LS3) from one another in the optical plane on the sample carrier (PTA, PTB), corresponding, respective focal points (FP1) arise in the optical plane on the sample carrier (PTA, PTB).

7. Apparatus for segmenting and separating patient samples (P) distributed over an area, having a sample carrier (PT, PT2, PTX, PTZ, PTA, PTB) suitable for receiving a liquid patient sample (P) and wherein the sample carrier has a material layer suitable for adsorbing the liquid patient sample, comprising

   - a holder (H) for holding the sample carrier (PT, PT2, PTX, PTZ, PTA, PTB) which has at least some of the patient sample (P) in or on a segment (S, S2, S11, S12) of the sample carrier (PT, PT2, PTX, PTZ, PTA, PTB),
   - a laser unit (LA, HL1, HL2, HL3) for producing a laser beam (LS, LS1, LS2, LS3),
   - at least one positioning means (PO) for altering a position of the laser beam (LS, LS1, LS2, LS3) in relation to the sample carrier (PT, PT2, PTX, PTZ, PTA, PTB),
   - at least one control unit (C) for driving the positioning means (PO) in such a way that the laser beam (LS, LS1, LS2, LS3) is guided along at least part of the boundary curve (R, R2, R11, R12) of the segment (S, S2, S11, S12) such that the segment (S, S2, S11, S12) is cut from the sample carrier (PT, PT2, PTX, PTZ, PTA, PTB),

   **characterized in that** the laser unit (LA, HL1, HL2, HL3) is a semiconductor laser (HL) that produces a minimum optical power of more than 1 watt and **in that** the laser beam (LS, LS1, LS2, LS3) has a wavelength in a range from 440 to 455 nm.

8. Apparatus according to Claim 7,

   wherein the sample carrier (PTZ, PTA, PTB) has cut-outs such that the segment (S, S11, S12) is connected to the remainder of the sample carrier (PTZ, PTA, PTB) by one or more webs (ST, ST11, ST12),
   **characterized in that** the control unit (C) is designed to drive the positioning means (PO) in such a way that the laser beam (LS, LS1, LS2, LS3) is guided along those portions of the boundary curve (R, R2, R11, R12) of the segment (S, S11, S12) where the webs (ST, ST11, ST12) are situated.

9. Apparatus according to Claim 7,
   further **characterized by**

   - an air delivery unit (LF) for feeding an airflow (LU) to an interface between the laser beam (LS, LS1, LS2, LS3) and the sample carrier (PT, PT2, PTX, PTZ, PTA, PTB)
   - and an exhaust air unit (AB) for aspirating at least some of the airflow (LU) away from the interface.

10. Apparatus according to Claim 7,
    further **characterized by**

    - a plurality of respective laser subunits (HL1, HL2, HL3) for producing respective laser beams (LS1, LS2, LS3),
    - and an optical unit (O) for steering the respective laser beams (LS1, LS2, LS3) to respective cutting points on the boundary curve (R11, R12),

further **characterized**

- **in that** the positioning means (PO) is suitable for altering respective positions of the respective laser beams (LS1, LS2, LS3) in relation to the sample carrier (PTA, PTB),
- and **in that** the control unit (C) is designed to drive the positioning means (PO) in such a way that the respective laser beams (LS1, LS2, LS3) are guided simultaneously along respective portions of the boundary curve (R11, R12) such that the segment (S11, S12) is cut from the sample carrier (PTA, PTB).

**11.** Apparatus according to Claim 10,

wherein the optical unit (O) is designed to steer the respective laser beams (LS1, LS2, LS3) to the respective, different cutting points on the boundary curve (R11, R12) in such a way that respective focal points (FP1) arise for the respective laser beams (LS1, LS2, LS3) in an optical plane that coincides with the sample carrier (PTA, PTB),
and that the optical unit is adjustable such that a distance of the respective cutting points of the respective laser beams (LS1, LS2, LS3) from one another in the optical plane on the sample carrier (PTA, PTB) can be set such that corresponding, respective focal points (FP2) also arise for different distances (AB1, AB2) of the respective cutting points in the optical plane on the sample carrier (PTA, PTB),
wherein, in particular, the control unit (C) is designed to drive the optical unit (O) by means of a control signal for altering or setting the distance of the respective cutting points in the optical plane on the sample carrier (PTA, PTB).

**12.** System comprising

- an apparatus (V) according to any one of Claims 7 to 11,
- wherein the sample carrier (PTZ, PTA, PTB) further has cut-outs (A) such that a segment (S, S11, S12) of the sample carrier is connected to the remainder of the sample carrier (PTZ, PTA, PTB) by one or more webs (ST, ST11, ST12).

**Revendications**

**1.** Procédé de segmentation et d'isolation d'échantillons de patient répartis de manière sensiblement bidimensionnelle, un porte-échantillon (PT, PT2, PTX, PTZ, PTA, PTB) étant apte à recevoir un échantillon de patient liquide (P), et le porte-échantillon comportant une couche de matériau qui est apte à adsorber l'échantillon de patient liquide, le procédé comprenant les étapes suivantes

- fournir le porte-échantillon qui comporte au moins une partie de l'échantillon de patient (P) dans ou sur un segment (S, S2, S11, S12),
- générer un faisceau laser (LS, LS1, LS2, LS3) au moyen d'une unité laser (LA, HL1, HL2, HL3),
- découper le segment (S, S2, S11, S12) du porte-échantillon (PT, PT2, PTX, PTZ, PTA, PTB) par guidage du faisceau laser (LS, LS1, LS2, LS3) le long d'au moins une partie d'un courbe de bord du segment,

**caractérisé en ce que** l'unité laser (LA, HL1, HL2, HL3) est un laser à semiconducteur (HL) qui génère une puissance optique minimale de plus de 1 watt, et **en ce que** le faisceau laser (LS, LS1, LS2, LS3) a une longueur d'onde dans une gamme (B) de 440 à 455 nm.

**2.** Procédé selon la revendication 1,
**caractérisé en ce que** le porte-échantillon (PTZ, PTA, PTB) comporte des évidements de sorte que le segment (S, S11, S12) soit relié au reste du porte-échantillon (PTZ, PTA, PTB) par une ou plusieurs nervures (ST, ST11, ST12), **caractérisé en outre par** l'étape de guidage du faisceau laser (LS, LS1, LS2, LS3) le long des zones partielles de la courbe de bord (R, R2, R11, R12) du segment (S, S11, S12) au niveau desquelles se trouvent les nervures (ST, ST11, ST12).

**3.** Procédé selon la revendication 1,
**caractérisé en ce que**

- le porte-échantillon (PT2) comporte une couche de film (TFL) qui est constante de manière sensiblement bidimensionnelle et qui comprend un film thermoplastique,

et le segment (S2) est formé à partir d'un segment de couche de matériau (STO2) qui est appliqué sur le film thermoplastique.

4. Procédé selon la revendication 1,
   **caractérisé en outre par** les étapes suivantes

   - amener un flux d'air (LU) à une interface du faisceau laser (LS, LS1, LS2, LS3) avec le porte-échantillon (PT, PT2, PTX, PTZ, PTA, PTB),
   - évacuer de l'interface par aspiration au moins une partie du flux d'air (LU).

5. Procédé selon la revendication 1,
   **caractérisé par** les étapes suivantes

   - générer plusieurs faisceaux laser respectifs (LS1, LS2, LS3) au moyen de sous-unités laser respectives (HL1, HL2, HL3),
   - diriger les faisceaux laser respectifs (LS2, LS2, LS3) vers différents points d'intersection respectifs sur la courbe de bord (R11, R12) au moyen d'une unité optique (O),
   - découper le segment (S11, S12) du porte-échantillon (PTA, PTB) tout en guidant les faisceaux laser respectifs (LS1, LS2, LS3) le long de sous-portion respectives de la courbe de bord (R11, R12) du segment (S11, S12).

6. Procédé selon la revendication 5,
   **caractérisé en outre par** les étapes suivantes

   - diriger, au moyen de l'unité optique, les faisceaux laser respectifs (LS1, LS2, LS3) vers les différents points d'intersection respectifs sur la courbe de bord (R11, R12) de manière à obtenir, pour les faisceaux laser respectifs (LS1, LS2, LS3), des points de focalisation respectifs (FP1) dans un plan optique qui coïncide avec le porte-échantillon (PTA, PTB),
   - et régler l'unité optique de manière à obtenir, pour une distance modifiée ou différente entre les points d'intersection respectifs des faisceaux laser respectifs (LS1, LS2, LS3) dans le plan optique sur le porte-échantillon (PTA, PTB), des points de focalisation respectifs (FP1) correspondants les uns aux autres dans le plan optique sur le porte-échantillon (PTA, PTB).

7. Dispositif de segmentation et d'isolation par découpe laser d'échantillons de patient (P) répartis de manière sensiblement bidimensionnelle,
   le dispositif comprenant un porte-échantillon (PT, PT2, PTX, PTZ, PTA, PTB) qui est apte à recevoir un échantillon de patient liquide (P), et le porte-échantillon comportant une couche de matériau qui est apte à adsorber l'échantillon de patient liquide, et comportant

   - un support (H) destiné à maintenir le porte-échantillon (PT, PT2, PTX, PTZ, PTA, PTB), qui comporte au moins une partie de l'échantillon de patient (P) dans ou sur un segment (S, S2, S11, S12) du porte-échantillon (PT, PT2, PTX, PTZ, PTA, PTB),
   - une unité laser (LA, HL1, HL2, HL3) destinée à générer un faisceau laser (LS, LS1, LS2, LS3),
   - au moins un moyen de positionnement (PO) destiné à modifier la position du faisceau laser (LS, LS1, LS2, LS3) par rapport au porte-échantillon (PT, PT2, PTX, PTZ, PTA, PTB),
   - au moins une unité de commande (C) destinée à commander le moyen de positionnement (PO) de manière à guider le faisceau laser (LS, LS1, LS2, LS3) le long d'au moins une partie d'une courbe de bord (R, R2, R11, R12) du segment (S, S2, S11, S12) afin de découper le segment (S, S2, S11, S12) du porte-échantillon (PT, PT2, PTX, PTZ, PTA, PTB),

   **caractérisé en ce que** l'unité laser (LA, HL1, HL2, HL3) est un laser à semiconducteur (HL) qui génère une puissance optique minimale de plus de 1 watt, et **en ce que** le faisceau laser (LS, LS1, LS2, LS3) a une longueur d'onde dans une gamme de 440 à 455 nm.

8. Dispositif selon la revendication 7,

le porte-échantillon (PTZ, PTA, PTB) comportant des évidements de manière à relier le segment (S, S11, S12) au reste du porte-échantillon (PTZ, PTA, PTB) par une ou plusieurs nervures (ST, ST11, ST12), **caractérisé en ce que** l'unité de commande (C) est conçue pour commander le moyen de positionnement (PO) de manière à guider le faisceau laser (LS, LS1, LS2, LS3) le long des zones partielles de la courbe de bord (R, R2, R11, R12) du segment (S, S11, S12) au niveau desquelles se trouvent les nervures (ST, ST11, ST12).

9. Dispositif selon la revendication 7,
   **caractérisé en outre par**

   - une unité de transporter d'air (LF) destinée à amener un flux d'air (LU) à une interface entre le faisceau laser (LS, LS1, LS2, LS3) et le porte-échantillon (PT, PT2, PTX, PTZ, PTA, PTB),
   - et une unité d'évacuation d'air (AB) destinée à évacuer de l'interface par aspiration au moins une partie du flux d'air (LU).

10. Dispositif selon la revendication 7,
    **caractérisé en outre par**

    - une pluralité de sous-unités laser respectives (HL1, HL2, HL3) destinées à générer des faisceaux laser respectifs (LS1, LS2, LS3),
    - ainsi qu'une unité optique (O) destinée à diriger les faisceaux laser respectifs (LS1, LS2, LS3) vers les points d'intersection respectifs sur la courbe de bord (R11, R12),

    **caractérisé en outre en ce que**

    - le moyen de positionnement (PO) est apte à modifier les positions respectives des faisceaux laser respectifs (LS1, LS2, LS3) par rapport au porte-échantillon (PTA, PTB),
    - et l'unité de commande (C) est conçue pour commander le moyen de positionnement (PO) de manière à guider les faisceaux laser respectifs (LS1, LS2, LS3) simultanément le long des sous-portions respectives de la courbe de bord (R11, R12) afin de découper le segment (S11, S12) du porte-échantillon (PTA, PTB).

11. Dispositif selon la revendication 10,

    l'unité optique (O) étant conçue pour diriger les faisceaux laser respectifs (LS1, LS2, LS3) vers les différents points d'intersection respectifs sur la courbe de bord (R11, R12) de façon à obtenir, pour les faisceaux laser respectifs (LS1, LS2, LS3), des points de focalisation respectifs (FP1) dans un plan optique qui coïncide avec le porte-échantillon (PTA, PTB),
    et l'unité optique pouvant être réglée de façon à pouvoir régler une distance entre les points d'intersection respectifs des faisceaux laser respectifs (LS1, LS2, LS3) dans le plan optique sur le porte-échantillon (PTA, PTB) afin d'obtenir, pour des distances différentes (AB1, AB2) des points d'intersection respectifs dans le plan optique sur le porte-échantillon (PTA, PTB), des points de focalisation respectifs correspondants (FP2),
    en particulier l'unité de commande (C) étant conçue pour commander l'unité optique (O) au moyen d'un signal de commande afin de modifier ou régler la distance entre les points d'intersection respectifs dans le plan optique sur le porte-échantillon (PTA, PTB).

12. Système comportant

    - un dispositif (V) selon l'une des revendications 7 à 11,
    - le porte-échantillon (PTZ, PTA, PTB) comportant également des évidements (A) de façon à relier un segment (S, S11, S12) du porte-échantillon au reste du porte-échantillon (PTZ, PTA, PTB) par une ou plusieurs nervures (ST, ST11, ST12).

**FIG. 1**

**FIG. 2**

**FIG. 3**

**FIG. 4**

Spektrale Remission

**FIG. 5**

**FIG. 6**

**FIG. 7**

EP 3 567 362 B1

**FIG. 8**

**FIG. 9**

**FIG. 10a**

HL1

HL2

HL3

9 mm

**FIG. 10b**

HL2

HL1

HL3

9 mm

**FIG. 11b**

**FIG. 11a**

**FIG. 12a**

```
┌──────────┐
│          │──── SC1
└──────────┘
     │
     ▼
┌──────────┐
│          │──── SC2
└──────────┘
     │
     ▼
┌──────────┐
│          │──── SC3
└──────────┘
```

**FIG. 12b**

```
┌──────────┐
│          │──── SC1
└──────────┘
     │
     ▼
┌──────────┐
│          │──── SC2A
└──────────┘
     │
     ▼
┌──────────┐
│          │──── SC3A
└──────────┘
     │
     ▼
┌──────────┐
│          │──── SC3B
└──────────┘
```

**FIG. 12c**

```
┌──────────┐
│          │──── SC0A
└──────────┘
     │
     ▼
┌──────────┐
│          │──── SC1
└──────────┘
     │
     ▼
┌──────────┐
│          │──── SC2A
└──────────┘
     │
     ▼
     ⋮
```

Fig. 13

SEG1　　SEG2　　SEG3

# Fig. 14a

# Fig. 14b

| Anti-Röteln-IgG | RE/ml | | |
|---|---|---|---|
| | Zange | gelasert | |
| Patient Nr. | | Stege | Kreise |
| 1 | 191 | 200 | 200 |
| 2 | 18 | 22 | 15 |
| 3 | 23 | 21 | 23 |
| 4 | 26 | 24 | 21 |
| 5 | - | 107 | 107 |

# Fig. 15a

**Vitamin D-ELISA**

# Fig. 15b

| Vitamin D | ng/ml | |
|---|---|---|
| Patient Nr. | Zange | Stege gelasert |
| 1 | 14 | 22 |
| 2 | 24 | 35 |
| 3 | 10 | 14 |
| 4 | 11 | 13 |
| 5 | 12 | 12 |

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20150040688 A1
- WO 2009068749 A2
- WO 0131317 A1